# EUROPEAN PATENT APPLICATION

(11) **EP 2 194 120 A1**
(43) Date of publication of application: **09.06.2010**
(21) Application number: 08291132.2
(22) Date of filing: 02.12.2008
(51) Int. Cl.: C12N 1/22, C12N 15/52, C12P 7/06, C12P 7/10, C12N 1/21, C12R 1/145

(54) **Bioprocessing ligno-cellulose into ethanol with recombinant clostridium**

(71) Applicant: Total S.A., 92400 Courbevoie (FR); Centre National de la Recherche Scientifique (CNRS), 75794 Paris Cedex 16 (FR); Université de la Méditerranée, 13284 Marseille Cédex 07 (FR); Université de Provence, 13331 Marseille Cédex 3 (FR); Institut National des Sciences Appliquées (INSA), 31077 Toulouse Cedex 4 (FR)
(72) Inventor: Girbal, Laurence, 31450 Pompertuzat (FR); Saint-Prix, Florence, 31400 Toulouse (FR); Mazzoli, Roberto, 10141 Torino (IT); Cesari, Mathieu, 31000 Toulouse (FR); Fierobe, Henri-Pierre, 13009 Marseille (FR); Mingardon, Florence, Pasadena, CA 91101 (US); Chanal-Vial, Angélique, 13012 Marseille (FR)
(74) Representative: Paemen, Liesbet R.J.

(57) **Abstract**

The present invention relates, *inter alia,* to recombinant Gram-positive *Clostridia* host cells for producing solvents, fuels and/or chemical intermediates, and preferably ethanol, from plant cell walls comprising: (a) at least one nucleic acid encoding a plant cell wall degrading enzyme, wherein the host cells produce and secrete the plant cell wall degrading enzyme, (b) at least one nucleic acid encoding an enzyme that converts pyruvate to acetaldehyde and at least one nucleic acid encoding an enzyme that converts acetaldehyde to ethanol wherein the host cell is capable of expressing said nucleic acid, and, (c) a mutation in at least one nucleic acid encoding for an enzyme in a metabolic pathway which produces a metabolite other than acetaldehyde from pyruvate or ethanol from acetaldehyde, such that the mutation results in a reduced production of the metabolite.

SEQUENCE LISTING

<110>  TOTAL S.A.
       TOTAL S.A. et al.

<120>  Bioprocessing ligno-cellulose into ethanol with recombinant
       clostridium

<130>  TOTAL-045-EP-PRIO

<160>  3

<170>  PatentIn version 3.3

<210>  1
<211>  1707
<212>  DNA
<213>  Artificial

<220>
<223>  Synthetic pdc

<400>  1
atgtcttata cagttggaac ttatttagca gaaagacttg ttcaaattgg attaaaacac      60

cattttgcag tagctggtga ttacaacctt gttcttttag ataatttact tcttaataaa     120

aatatggaac aggtttactg ctgcaatgaa cttaattgtg gattctcagc agaaggatat     180

gcaagagcta aaggtgctgc tgctgctgtt gtaacatact cagtaggagc attatcagct     240

tttgatgcaa ttggaggtgc atatgctgaa aacttacctg ttattcttat atcaggagct     300

ccaaataata acgatcatgc tgcaggacac gtacttcatc atgcattagg taagacagat     360

taccactatc agttagaaat ggcaaaaaat ataactgctg ctgctgaagc aatatataca     420

ccagaagaag ctccagctaa aatagatcat gttataaaaa ctgctttaag agaaaagaag     480

ccagtttacc ttgaaatagc ttgcaatata gctagtatgc catgcgcagc tccaggacca     540

gcatctgctc tttttaatga tgaagcttct gatgaagctt cacttaacgc agctgttgaa     600

gaaactctta aattcattgc taatagagat aaagtagctg ttcttgttgg atctaagctt     660

agagcagcag gagcagaaga agcagctgtt aagttcgcag atgctttagg aggagcagtt     720

gcaacaatgg ctgcagcaaa atcattcttt ccagaagaaa atccacacta tataggtaca     780

tcttggggag aagtatcata tccaggagta gaaaagacaa tgaaggaagc tgatgcagta     840

attgcacttg cacctgtttt taatgattat agtactactg gttggacaga tatacctgat     900

cctaaaaaat tagtacttgc tgaaccaaga agtgtagttg ttaatggaat aagattccca     960

tcagttcatc ttaaggatta cttaacaaga cttgcacaaa aagtttctaa aaagactggt    1020

gcacttgatt tctttaagtc acttaatgca ggagaactta agaaagctgc tccagcagat    1080

ccatctgctc cattagttaa tgctgaaatt gcaagacaag ttgaagctct tcttactcct    1140

aatacaacag ttattgctga aacaggagat tcttggttca acgctcagag aatgaagtta    1200

ccaaatggag caagagttga atacgaaatg caatggggtc atattggatg gtcagtacct    1260

gcagcattcg gttatgcagt tggtgcacct gaaagaagaa atattcttat ggttggagat    1320

ggatcatttc aattaacagc acaagaagtt gctcaaatgg ttagacttaa gcttccagta    1380

attatattcc ttataaacaa ttatggatat acaatagaag taatgattca cgatggacct    1440

tataataata taaaaaattg ggattatgca ggtttaatgg aagtatttaa tggtaatgga 1500

ggttatgatt caggagctgg taaaggatta aaagcaaaga ctggtggtga acttgcagaa 1560

gctataaagg ttgcacttgc aaatacagat ggaccaactt taatagaatg tttcatagga 1620

agagaagatt gtacagaaga acttgttaaa tggggaaaaa gagttgcagc agctaattca 1680

agaaaaccag taaataagct tttataa 1707

<210> 2
<211> 50
<212> DNA
<213> Artificial

<220>
<223> Primer sequence ADH_D

<400> 2
aaaaggcgcc gggaggataa acatgtctat cccagaaact caaaaaggtg 50

<210> 3
<211> 40
<212> DNA
<213> Artificial

<220>
<223> Primer sequence ADH_R

<400> 3
aaaaggcgcc ttatttagaa gtgtcaacaa cgtatctacc 40

## Description

### Technical Field of invention

The invention relates to the field of industrial microbiology and the production of alcohol through industrial fermentation with a recombinant microorganism. More specifically, solvents, fuels and/or chemical intermediates, such as for instance ethanol are/is produced through the fermentation of lignocellulosic materials using a recombinant *Clostridium* strain.

### Background

Ethanol is an excellent transportation fuel which is in some aspects superior to petroleum-based fuels. Ethanol has a higher octane rating and can be burned more cleanly and with higher efficiency. It is particularly beneficial with respect to low CO₂ output into the atmosphere. Furthermore, the low volatility and the photochemical reactivity of ethanol reduce smog formation and only low levels of smog-producing compounds are formed by its combustion. Furthermore, the combustion products of ethanol show similar characteristics. A good engine performance is obtained due to the high heat of vaporization, the high octane rating and the low flame temperature.

Currently ethanol is mainly produced through yeast fermentation of hexose sugars derived from corn starch or cane syrup. However, these are relatively expensive sources of biomass sugars and have competing value as foods. Starches and sugars represent only a fraction of the total carbohydrates in plants.

Therefore the fermentation of lignocellulosic biomass to ethanol is an attractive route to energy that supplements the depleting stores of fossil fuels. Lignocellulosic biomass is a carbon-neutral source of energy, since it comes from dead plants, which means that the combustion of ethanol produced from lignocelluloses will produce substantially no net carbon dioxide in the earth's atmosphere. Also, biomass is readily available, and the fermentation of lignocelluloses provides an attractive way to dispose of many industrial and agricultural waste products. Furthermore lignocellulosic biomass is a very abundant renewable natural resource and substrate available for conversion to fuels. It is inexpensive, plentiful and renewable. Lignocellulose is primarily a mixture of cellulose, hemicellulose, and lignin typically in the range of approximately 35 to 50 %, 20 to 35 % and 5 to 30% of plant dry weight, respectively. Cellulose is a homopolymer of glucose, while hemicellulose is a more complex heteropolymer comprised not only of xylose, which is its primary constituent, but also of significant amounts of arabinose, mannose, glucose, and galactose. Hydrolysis of these polymers releases a mixture of neutral sugars which include glucose, xylose, mannose, galactose, and arabinose.

Process designs for biologically converting cellulosic materials nearly always include a pretreatment step to convert lignocellulosic biomass from its native form in which is recalcitrant to cellulase enzyme systems, into a form for which enzymatic hydrolysis is effective. Four biologically mediated events occur in the course of converting biomass into fuels and chemicals in processes featuring enzymatic hydrolysis: (i) cellulase production, (ii) hydrolysis of cellulose and (if present) other insoluble polysaccharides, (iii) fermentation of soluble cellulose hydrolysis products and (iv) fermentation of soluble hemicellulose hydrolysis products. This type of bioprocessing is however very cumbersome and complicated due to the many process steps required in these process designs. Consequently these type of processes are difficult to control and very time consuming.

Solventogenic bacteria are bacteria capable of converting sugars into solvents. *Clostridium acetobutylicum* for instance enables the conversion of sugars into ethanol, acetone and butanol during the well-known acetone-butanol-ethanol (ABE) fermentation. The production of acetone and butanol by means of *C. acetobutylicum* was one of the first large-scale industrial fermentation processes to be developed. In a typical ABE fermentation, butyric, propionic, lactic and acetic acids are first produced by *C. acetobutylicum,* the culture pH drops and undergoes a metabolic "butterfly" shift, and 1-butanol, acetone, isopropanol and ethanol are then formed. However, in ABE fermentations, the ethanol yield from glucose is low, typically around 0.1 mol ethanol per mol glucose and rarely exceeding 0.5 mol ethanol per mol glucose. Consequently, the ethanol yield in conventional ABE fermentations is usually lower than 0.25 mol ethanol per mol glucose. Theoretically an ethanol maximal yield of 2 mol ethanol per mol glucose can be obtained from an ABE fermentation process.

Also, ABE fermentations have been quite complicated and difficult to control. The use of ABE fermentation has declined continuously since the 1950s, and almost all acetone, butanol and ethanol are now produced through petrochemical routes.

Another disadvantage is that the process produces significant amounts of acetone which is not useful as a gasoline additive.

### Summary of the invention

There is a need in the art for processes permitting the conversion of lignocellulose into ethanol, optimally which incorporate pre-treatment of lignocellulose, such as to overcome at least some disadvantages of prior-art processes.

The invention provides recombinant micro-organisms having an engineered lignocellulose hydrolysis pathway and an engineered biosynthesis pathway for one or more solvents, fuels and/or chemical intermediates. The recombinant micro-organisms therefore provide a combination of the properties related to both lignocellulosic biomass utilization and synthesis of solvents, fuels and/or chemical intermediates, such as ethanol. The engineered micro-organism may be used for the commercial production of solvents, fuels and/or chemical intermediates such as ethanol from lignocellulosic materials.

The present invention provides consolidated bioprocessing: cellulase production, hydrolysis, and fermentation of products of both cellulose and hemicellulose hydrolysis are accomplished in a single process step. Thereto, the present invention provides a microbial culture that combines properties related to both lignocellulosic biomass utilization and formation of solvents, fuels and/or chemical intermediates, such as ethanol.

The present application relates to the metabolic engineering of microorganisms, more particularly of solventogenic microorganisms. Providing a solventogenic microorganism with a plant cell wall degrading enzyme, such as a cellulase, allows the direct production of useful solvents, fuels and/or chemical intermediates such as ethanol from cellulose-containing substrates. Additionally, the present application provides in the engineering of the solventogenic metabolism of a microorganism, thereby providing an increased production yield of desirable solvents, fuels and/or chemical intermediates, such as ethanol. Different aspects of the metabolic engineering of solventogenic microorganisms of the present invention allow the combination of unique properties that on the one hand enable the use of lignocellulosic materials as a source for its metabolism and on the other hand provide an increased production yield of useful solvents, fuels and/or chemical intermediates, such as ethanol. In particular embodiments, these different aspects are applied to one and the same microorganism, more particularly to a single solventogenic microorganism.

In particular embodiments of the aspects described herein bellow, recombinant Gram-positive *Clostridia* host cells are used, more particularly a *Clostridium* species selected from the group comprising or consisting of *Clostridium acetobutylicum* and *C. beijerinckii* and more preferably the recombinant host cell is a *Clostridium acetobutylicum.*

In one aspect the present invention relates to recombinant Gram-positive *Clostridia* host cells comprising:
(a) at least one heterologous nucleic acid encoding a plant cell wall degrading enzyme and/or a cellulosomal scaffoldin protein, wherein the host cell is capable of expressing said nucleic acids and of producing and secreting said plant cell wall degrading enzyme or cellulosomal scaffoldin protein
(b) at least one nucleic acid encoding an enzyme that converts pyruvate to acetaldehyde and optionally at least one heterologous nucleic acid encoding an enzyme that converts acetaldehyde to ethanol wherein said host cell is capable of expressing said nucleic acid; and/or
(c) a mutation in at least one nucleic acid encoding for an enzyme in a metabolic pathway in said host cell, wherein said pathway produces a metabolite other than acetaldehyde from pyruvate or ethanol from acetaldehyde, and wherein said mutation results in a reduced production of said metabolite.

The recombinant host cells of the invention provide specific advantages for use in the production of solvents, fuels and/or chemical intermediates. Each of the features (a), (b) and (c) can be introduced into a micro-organism, most particularly into the same microorganism.

In a further aspect, the present invention relates to recombinant Gram-positive *Clostridia* host cells for producing solvents, fuels and/or chemical intermediates, and preferably ethanol, from plant cell walls, which host cells comprise:
(1) at least one nucleic acid encoding an enzyme that converts pyruvate to acetaldehyde in association or not with at least one heterologous nucleic acid encoding an enzyme that converts acetaldehyde to ethanol wherein said host cell is capable of expressing said nucleic acid, and,
(2) a mutation in at least one nucleic acid encoding for an enzyme in a metabolic pathway in said host cell, wherein said pathway produces a metabolite other than acetaldehyde from pyruvate or ethanol from acetaldehyde, and wherein said mutation results in a reduced production of said metabolite.

In a further aspect, the present invention relates to recombinant Gram-positive *Clostridia* host cells for producing solvents, fuels and/or chemical intermediates, and preferably ethanol, from plant cell walls which host cells comprise:
(a) at least one nucleic acid encoding a plant cell wall degrading enzyme and/or a cellulosomal scaffoldin protein, wherein the host cell is capable of expressing said nucleic acid and of producing and secreting said plant cell wall degrading enzyme or cellulosomal scaffoldin protein, and
(b) at least one nucleic acid encoding an enzyme that converts pyruvate to acetaldehyde in association or not with at least one heterologous nucleic acid encoding an enzyme that converts acetaldehyde to ethanol wherein said host cell is capable of expressing said nucleic acid, and,
(c) a mutation in at least one nucleic acid encoding for an enzyme in a metabolic pathway in said host cell, wherein said pathway produces a metabolite other than acetaldehyde from pyruvate or ethanol from acetaldehyde, and wherein said mutation results in a reduced production of said metabolite.

The metabolic engineering of the solventogenic microorganism according to these aspects of the present invention on the one hand enables the use of lignocellulosic materials as a source for its metabolism and on the other hand ensures an increased production yield of desirable solvents, fuels and/or chemical intermediates, such as ethanol.

In yet a further aspect, the present invention provides recombinant Gram-positive microorganisms, more particularly Gram-positive *Clostridia* host cells comprising at least one heterologous nucleic acid encoding a plant cell wall degrading enzyme or encoding a cellulosomal scaffoldin, wherein the host cell is capable of expressing said nucleic acid and of producing and secreting said plant cell wall degrading enzymes or cellulosomal scaffoldin.

In particular embodiments recombinant *Clostridia* host cells are provided comprising at least one heterologous nucleic acid encoding one or more plant cell wall degrading enzymes, wherein these cell wall degrading enzymes are cellulases appended with appropriate dockerin domains or modules and further comprising at least one heterologous nucleic acid encoding a wild-type or hybrid scaffoldin bearing the cognate cohesin domains or modules, whereby the host cells are capable of expressing these nucleic acids and produce extracellularly a cellulosome composed of the expressed cellulases bound to the expressed scaffoldin.

In particular embodiments of the aspects described herein, the recombinant *Clostridia* host cells comprise at least one heterologous nucleic acid encoding one or more plant cell wall degrading enzymes, wherein said cell wall degrading enzymes are cellulases. More particularly, the plant cell wall degrading enzymes envisaged in the context of this invention are endoglucanases, exoglucanases and/or endo-processive cellulases.

In further particular embodiments, host cells are provided as described hereinabove wherein the plant cell wall degrading enzymes are cellulases selected from the group consisting of
a. Cellulases of *C. cellulolyticum,* and preferably selected from the group comprising Cel48F, Cel9G, Cel9R, Cel9P, Cel9E, Cel9H, Cel93, Cel9M, Cel8C, Cel440, Cel5N and Cel5A, and functional fragments and/or functional variants of any of said cellulases; and
b. Cellulases of *S. degradans* strain 2-40 and preferably selected from the group comprising Cel9A, Cel9B, Cel5J, Cel5I, Cel5F, Cel5H, Cel5D, Cel5B, Cel9G, Cel5E, Cel5A, Cel5C and Cel6A and functional fragments and/or functional variants of any of said cellulases.

In particular embodiments, host cells are provided wherein the plant cell wall degrading enzymes are part of a cellulolytic complex. This is achieved either through the introduction of nucleic acids which encode the cellulolytic complex or through introduction of a cellulosomal scaffolding protein which allows binding of one or more plant cell wall degrading enzymes.

In further particular embodiments, the cellulolytic complex is composed of a scaffoldin protein preferably selected from the group comprising CipC of *C. cellulolyticum,* CipA of *C. thermocellum,* CbpA of *C. cellulovorans,* CipA of *C. acetobutylicum* and CipA of *C. josui,* and at least two cellulases which are appended to said scaffoldin protein with appropriate dockerins or operably linked to said scaffolding protein. As detailed herein, in particular embodiments host cells are provided which comprise one or more heterologous nucleic acids encoding cellulases appended to appropriate dockerin domains and one or more heterologous nucleic acids encoding a scaffoldin (which is either hybrid or wild-type) bearing the cognate cohesion domains. Upon expression of these heterologous nucleic acids, the cellulase(s) and scaffoldin combine into a cellulosome.

In yet a further aspect of the present invention host cells are provided which comprise a recombinant solventogenic metabolism adapted to increase the production of solvents, fuels and/or chemical intermediates. More preferably host cells of the present invention comprise a recombinant solventogenic metabolism adapted to increase ethanol production.

In particular embodiments of the aspects described above the recombinant solventogenic metabolism of a host cell of the present invention comprises at least one nucleic acid encoding an enzyme that converts pyruvate to acetaldehyde and at least one nucleic acid encoding an enzyme that converts acetaldehyde to ethanol wherein said host cell is capable of expressing said nucleic acid. Preferably the nucleic acid encoding an enzyme that converts pyruvate to acetaldehyde is a heterologous nucleic acid whereas the nucleic acid encoding an enzyme that converts acetaldehyde to ethanol can either be an endogenous or a heterologous nucleic acid.

In further particular embodiments host cells are provided, wherein the recombinant solventogenic metabolism comprises at least one nucleic acid encoding an enzyme that converts pyruvate to acetaldehyde in association or not with at least one heterologous nucleic acid encoding an enzyme that converts acetaldehyde to ethanol wherein said host cells are capable of expressing said nucleic acid(s). More particularly, host cells are provided, wherein the nucleic acid encoding an enzyme that converts pyruvate to acetaldehyde is pyruvate decarboxylase (*pdc*), more particularly pyruvate decarboxylase nucleic acid obtained from *Zymomonas mobilis.*

In particular embodiments, host cells of the invention comprise a heterologous nucleic acid encoding an enzyme that converts acetaldehyde to ethanol which is an alcohol dehydrogenase (adh). More particularly the heterologous alcohol dehydrogenase is obtained from *Saccharomyces cerevisiae.*

In further particular embodiments, the recombinant solventogenic metabolism of host cells according to the present invention comprises a mutation in at least one nucleic acid encoding for an enzyme in a metabolic pathway in said host cell, wherein said pathway produces a metabolite other than acetaldehyde from pyruvate or ethanol from acetaldehyde, and wherein the mutation results in a reduced production of said metabolite.

In more particular embodiments the mutation is a mutation in at least one nucleic acid encoding an enzyme chosen from the group comprising pyruvate ferredoxin oxidoreductase (*pfor*), phosphotransacetylase (*pta*), acetate kinase (*ak*), coenzym A transferase (*ctfAB*)*,* acetoacetate decarboxylase (*adc*), phosphotransbutyrylase (*ptb*)*,* butyrate kinase (*bk*), lactate dehydrogenase (*ldh*), thiolase (*thl*), β-hydroxybutyryl coenzyme A dehydrogenase (*hbd*), crotonase (*crt*), butyryl coenzyme A dehydrogenase (*bcd*), bifunctional butyraldehyde-butanol dehydrogenase (*aad*/*adhE, adhE2*) and/or butanol dehydrogenase (*bdhAB*) and preferably chosen from the group consisting of pyruvate ferredoxin oxidoreductase (*pfor*)*,* phosphotransacetylase (*pta*)*,* acetate kinase (*ak*)*,* coenzym A transferase *(*c*tfAB*)*,* acetoacetate decarboxylase (*adc*), phosphotransbutyrylase (*ptb*)*,* butyrate kinase (*bk*), lactate dehydrogenase (*Idh*)*,* bifunctional butyraldehyde-butanol dehydrogenase (*aad*/*adhE, adhE2)* and/or butanol dehydrogenase *(bdhAB*).

In particular embodiments host cells are provided comprising a mutation in at least one nucleic acid encoding an enzyme in the metabolic pathway that converts pyruvate to lactate, more particularly comprising a mutation in the nucleic acid encoding lactate dehydrogenase (Idh).

In further particular embodiments host cells are provided comprising a mutation in at least one nucleic acid encoding an enzyme in the metabolic pathway that converts pyruvate to acetyl coenzyme A, more particularly comprising a mutation in the nucleic acid encoding pyruvate ferredoxin oxidoreductase (*pfor*)*,*

In yet further particular embodiments host cells are provided comprising a mutation in at least one nucleic acid encoding an enzyme in the metabolic pathway that converts acetyl coenzyme A to acetate, more particularly comprising a mutation in at least one nucleic acid encoding an enzyme chosen from the group comprising phosphotransacetylase (pta) and acetate kinase (ak).

In yet further particular embodiments host cells are provided comprising a mutation in at least one nucleic acid encoding an enzyme in the metabolic pathway that converts acetoacetyl coenzyme A to acetone, more particularly in at least one nucleic acid chosen from the group comprising coenzym A transferase (*ctfAB*) and acetoacetate decarboxylase (*adc*).

In yet further particular embodiments host cells are provided comprising, a mutation in at least one nucleic acid encoding an enzyme in the metabolic pathway that converts butyryl coenzyme A to butyrate, more particularly in at least one nucleic acid encoding an enzyme chosen from the group comprising phosphotransbutyrylase (*ptb*) and butyrate kinase (*bk*).

In particular embodiments of the different aspects described above, the recombinant host *Clostridia* host cell species is selected from the group comprising *Clostridium acetobutylicum and C. beijerinckii.* The generation of solventogenic Clostridium species, more particularly such as *Clostridium acetobutylicum* with increased production of solvents, fuels and/or chemical intermediates provides important advantages for industrial production of these substances.

In another aspect, the present invention relates to a method for producing solvents, fuels and/or chemical intermediates from biomass comprising plant cell walls comprising contacting said biomass with a host cell of the present invention, wherein the solvent, fuel and/or chemical intermediate is preferably ethanol.

In particular embodiments methods are provided which comprise contacting biomass with a recombinant host cell according to particular embodiments of the recombinant host cells of the invention, wherein the recombinant host cell provides cell wall degrading enzymes for the degradation of biomass.

A further aspect provides methods for degradation of biomass and production of solvents, fuels and/or chemical intermediates, and preferably ethanol, using a recombinant host cell according one or more aspects of the invention, wherein said recombinant host cell provides cell wall degrading enzymes for the degradation of biomass and a recombinant solventogenic metabolism.

In particular embodiments, methods are provided wherein the production of solvents, fuels and/or chemical intermediates is done under anaerobic conditions.

The present invention further relates to the use of a host cell comprising at least one nucleic acid encoding a plant cell wall degrading enzyme according to the present invention, wherein said host cell can be used for the degradation of biomass.

The present invention further relates to the use of one host cell incorporating the different aspects of the present invention, wherein said host cell can be used for the production of solvents, fuels and/or chemical intermediates, such as ethanol.

### Brief description of the Figures

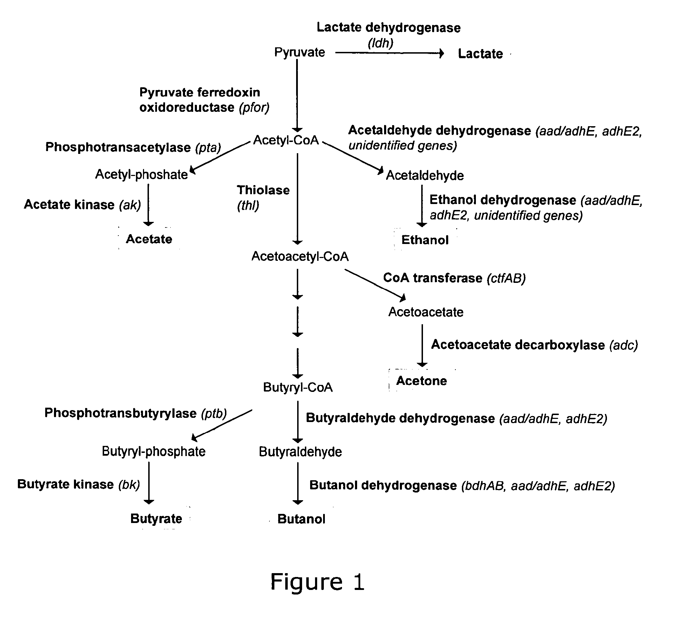

- Figure 1: illustrates an embodiment of an ABE fermentation pathway.

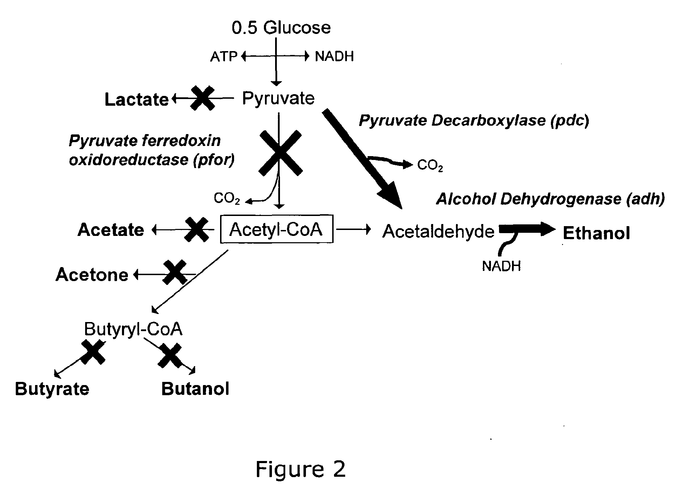

- Figure 2: illustrates an embodiment of an engineered ABE fermentation pathway.
- Figure 3a: illustrates an embodiment of a minicellulosome (1: family3a CBM; 2, catalytic modules; 3, cohesin modules; 4, dockerin modules; 5, X module; 6, linkers.
- Figure 3b: illustrates an embodiment of a Hybrid minicellulosome (legend same as figure 3A except that 3, 3' and 3" symbolise cohesins of different species and 4, 4', and 4" designate dockerins from different species.
- Figure 4: illustrates an embodiment of a covalent cellulosome complex (legend same as figure 3A).

### Detailed description of the invention

Before the present methods, cells and systems used in the invention are described, it is to be understood that this invention is not limited to particular methods, cells or systems described, as such methods, cells and systems may, of course, vary. It is also to be understood that the terminology used herein is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

As used herein, the singular forms "a", "an", and "the" include both singular and plural referents unless the context clearly dictates otherwise. By way of example, "a cell" refers to one or more than one cells.

The terms "comprising", "comprises" and "comprised of" as used herein are synonymous with "including", "includes" or "containing", "contains", and are inclusive or open-ended and do not exclude additional, non-recited members, elements or method steps.

The term "about" as used herein when referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less from the specified value, insofar such variations are appropriate to perform in the disclosed invention.

The recitation of numerical ranges by endpoints includes all numbers and fractions subsumed within that range, as well as the recited endpoints.

The term "nucleic acid" is intended to include nucleic acid molecules, e.g., polynucleotide sequences which include an open reading frame encoding a polypeptide, and can further include non-coding regulatory sequences, and introns. Nucleic acid molecules in accordance with the invention include DNA molecules (e.g., linear, circular, cDNA or chromosomal DNA) and RNA molecules (e.g., tRNA, rRNA, mRNA) and analogs of the DNA or RNA generated using nucleotide analogs. The nucleic acid molecule can be single-stranded or double-stranded, but advantageously is double-stranded DNA.

The terms "recombinant nucleic acid" or "recombinant nucleic acid molecule" as used herein generally refer to nucleic acid molecules (such as, e.g., DNA, cDNA or RNA molecules) comprising segments generated and/or joined together using recombinant DNA technology, such as for example molecular cloning and nucleic acid amplification. Usually, a recombinant nucleic acid molecule may comprise one or more non-naturally occurring sequences, and/or may comprise segments corresponding to naturally occurring sequences that are not positioned as they would be positioned in a source genome which has not been modified. When a recombinant nucleic acid molecule replicates in the host organism into which it has been introduced, the progeny nucleic acid molecule(s) are also encompassed within the term "recombinant nucleic acid molecule".

In particular embodiments, a recombinant nucleic acid molecule can be stably integrated into the genome of a host organism, such as for example integrated at one or more random positions or integrated in a targeted manner, such as, e.g., by means of homologous recombination, or the recombinant nucleic acid molecule can be present as or comprised within an extra-chromosomal element, wherein the latter may be auto-replicating.

The term "recombinant polypeptide" as used herein refers to a polypeptide or protein produced by a host organism through the expression of a recombinant nucleic acid molecule, which has been introduced into said host organism or an ancestor thereof, and which comprises a sequence encoding said polypeptide or protein.

Standard reference works setting forth the general principles of recombinant DNA technology include Molecular Cloning: A Laboratory Manual, 2nd ed., vol. 1-3, ed. Sambrook el al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates); Innis et al., PCR Protocols: A Guide to Methods and Applications, Academic Press: San Diego, 1990. General principles of microbiology are set forth, for example, in Davis, B. D. et al., Microbiology, 3d edition, Harper & Row, publishers, Philadelphia, Pa. (1980).

The term "transformation" encompasses the introduction or transfer of a foreign nucleic acid such as a recombinant nucleic acid into a host organism, microorganism or cell. The so-introduced nucleic acid may be preferably maintained throughout the further growth and cell division of said host organism, microorganism or cell. Any conventional gene transfer methods may be used to achieve transformation, such as without limitation electroporation, chemical transformation, lipofection, virus- or bacteriophage-mediated transfection, etc.

As used herein the terms "recombinant host cell", "recombinant microorganism" and the like, are intended to include cells encompass microorganisms or cells into which a recombinant nucleic acid molecule has been introduced, as well as the recombinant progeny of such microorganism or cells. The cell can be a microorganism or a higher eukaryotic cell. The term is intended to include progeny of the cell originally transfected. In some embodiments, the cell is a bacterial cell, e.g., a Gram-positive bacterial cell or a Gram-negative bacterial cell.

The terms "Gram-negative bacteria" and "Gram-positive bacteria" are intended to include the definitions of these terms as recognized in the state of the art. Gram-negative bacteria include, for example, the family Enterobacteriaceae which comprises *Escherichia, Shigella, Citrobacter, Salmonella, Klebsiella, Enterobacter, Erwinia, Kluyvera, Serratia, Cedecea, Morganella, Hafnia, Edwardsiella, Providencia, Proteus* and/or *Yersinia.* Other Gram-negative bacteria include, but are not limited to, *Acinetobacter, Gluconobacter, Geobacter* and *Shewanella.* Gram-positive bacteria include, but are not limited to, *Bacillus, Geobacillus, Clostridium, Streptococcus, Cellulomonas, Corynebacterium, Lactobacillis, Lactococcus, Oenococcus* and/or *Eubacterium.* Preferably the recombinant hosts are *Clostridium* cells and more preferably *Clostridium acetobutylicum* cells.

The terms "solventogenic" or "solvent-producing" have their art-established meaning and in particular denote the ability of microorganisms such as bacteria (i.e., solventogenic bacteria) to produce one or more non-gaseous organic liquids or solvents, such as inter alia ethanol, acetone, butanol, isopropanol, propanol, 1,2 propanediol, propionic acid, butyric acid, ether or glycerine, from a carbohydrate source such as for example hexoses, pentoses or oligosaccharides. In particular, the term encompasses naturally occurring solventogenic organisms, solventogenic organisms with naturally occurring or induced mutations, and solventogenic organisms which have been genetically modified. The term "solventogenic metabolism" when referring to a microorganism as used herein refers to the consecutive steps and/or enzymes which in that organism allow the production of solvents (and/or fuels and/or chemical intermediates, where appropriate).

The terms "ethanologenic" or "ethanol-producing" is intended to denote the ability of microorganisms such as bacteria (i.e., ethanologenic bacteria) to produce at least or preferably mainly ethanol from a carbohydrate source such as for example hexose, pentose or oligosaccharides, more preferably to produce ethanol from carbohydrates as the most abundant non-gaseous fermentation product. In particular, the term encompasses naturally occurring ethanologenic organisms, ethanologenic organisms with naturally occurring or induced mutations, and ethanologenic organisms which have been genetically modified.

The term "heterologous polynucleotide sequence" may refer to a polynucleotide sequence that as such is not naturally occurring in an organism, e.g., a sequence that is introduced into the organism. A heterologous polynucleotide sequence may be derived from any source, e.g., eukaryotes, prokaryotes, viruses, and/or synthetic polynucleotide fragments.

In order to optimize translation efficiency, modified nucleic acid sequences may be used, the sequence design being based on the codon usage of the host microorganism.

A "gene" as used herein, is a nucleic acid enabling the synthesis of an enzyme or other polypeptide molecule. The nucleic acid can comprise coding sequences, for example, a contiguous open reading frame (ORF) which encodes a polypeptide, or can itself be functional in the organism. A gene in an organism can be clustered in an operon, as defined herein, wherein the operon is separated from other genes and/or operons by intergenic DNA. Individual genes contained within an operon can overlap without intergenic DNA between the individual genes.

The term "homolog", as used herein, includes a polypeptide or polypeptide sharing at least about 30-35%, preferably at least about 35-40%, more preferably at least about 40-50%, and even more preferably at least about 60%, 70%, 80%, 90% or more identity with the amino acid sequence of a wild-type polypeptide or polypeptide described herein and having a substantially equivalent functional or biological activity as the wild-type polypeptide or polypeptide. Thus, the term "homolog" in intended to encompass "functional variants" as well as "orthologs".

The term "mutation", as used herein, is intended to refer to a relatively permanent change in the hereditary material of an organism involving either an aberration in one or more chromosomes, or a change in the DNA sequence. A mutation, as used herein, includes a change in a DNA sequence created either by deletion or insertion of a DNA sequence, by a change in one or more bases {e.g. , a point mutation), by duplication, by missense, by frameshift, by repeat or by nonsense mutation. Methods of creating insertion, deletion, and base change mutations are known in the art.

By "encoding" is meant that a nucleic acid sequence or part(s) thereof corresponds, by virtue of the genetic code of an organism in question to a particular amino acid sequence, e.g., the amino acid sequence of a desired polypeptide or protein. By means of example, nucleic acids "encoding" a particular polypeptide or protein may encompass genomic, hnRNA, pre-mRNA, mRNA, cDNA, recombinant or synthetic nucleic acids.

Preferably, a nucleic acid encoding a particular polypeptide or protein may comprise an open reading frame (ORF) encoding said polypeptide or protein. An "open reading frame" or "ORF" refers to a succession of coding nucleotide triplets (codons) starting with a translation initiation codon and closing with a translation termination codon known per se, and not containing any internal in-frame translation termination codon, and potentially capable of encoding a polypeptide. Hence, the term may be synonymous with "coding sequence" as used in the art.

In an embodiment, the nucleic acid sequence or ORF encoding the present polypeptide(s) may be codon optimised as known per se for expression in a particular organism, e.g., microorganism, more particularly a bacterium of interest. Codon usage bias and codon frequencies from various organisms are available, for example via the Codon Usage Database (http://www.kazusa.or.jp/codon/) described by Nakamura et al. 2000 (Nucl Acids Res 28: 292).

Expression of polypeptides of interest in recombinant microorganisms as taught herein can be achieved through operably linking the nucleic acid sequences or ORF(s) which encode said polypeptides with regulatory sequences allowing for expression in said microorganisms.

An "operable linkage" is a linkage in which regulatory nucleic acid sequences and sequences sought to be expressed are connected in such a way as to permit said expression. For example, sequences, such as, e.g., a promoter and an ORF, may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence.

The precise nature of regulatory sequences or elements required for expression may vary between organisms, but typically include a promoter and a transcription terminator, and optionally an enhancer.

Reference to a "promoter" or "enhancer" is to be taken in its broadest context and includes transcriptional regulatory sequences required for accurate transcription initiation and where applicable accurate spatial and/or temporal control of gene expression or its response to, e.g., external stimuli. More particularly, "promoter" may depict a region on a nucleic acid molecule, preferably DNA molecule, to which an RNA polymerase binds and initiates transcription. A promoter is preferably, but not necessarily, positioned upstream, i.e., 5', of the sequence the transcription of which it controls. Typically, in prokaryotes a promoter region may contain both the promoter per se and sequences which, when transcribed into RNA, will signal the initiation of protein synthesis (e.g., Shine-Dalgarno sequence).

As used herein the terms "lignocellulosic biomass", "lignocellulose" and the like, refer to plant biomass that is composed of cellulose, hemicellulose and lignin. The cellulose and hemicellulose carbohydrate polymers are tightly bound to the lignin, by hydrogen and covalent bonds. Lignocellulosic biomass can be obtained from a variety of sources including, but not limited to, wood residues such as sawmill and paper mill discards, municipal paper waste, agricultural residues such as corn stover and sugarcane bagasse, and dedicated energy crops such as crops composed of fast growing, tall, woody grasses.

The terms "lignocellulose degrading enzymes", "polysaccharase", "cellulase" "cell wall degrading enzymes" or "glucanase" are used interchangeably herein and are intended to include a polypeptide capable of catalyzing the degradation or depolymerization of any linked sugar moiety, e.g., disaccharides, trisaccharides, oligosaccharides, including complex carbohydrates, also referred to herein as complex sugars, e.g., cellooligosaccharide and lignocellulose, which comprise cellulose, hemicellulose, and pectin. The terms are intended to include cellulases such as glucanases, including, preferably, endoglucanases but also including, e.g., exoglucanase, [beta]-glucosidase, cellobiohydrolase, endo-1,4-[beta]-xylanase, [beta]-xylosidase, [alpha]-glucuronidase, [alpha]-L-arabinofuranosidase, acetylesterase, acetylxylanesterase, [alpha]-amylase, [beta]-amylase, glucoamylase, pullulanase, [beta]-glucanase, hemicellulase, arabinosidase, mannanase, pectin hydrolase, pectate lyase, or a combination of any of these glucanases.

The terms "fermentation" and "fermenting" are intended to include the degradation or depolymerization of a complex sugar and bioconversion of that sugar residue into ethanol, other minor fermentation products. The terms are intended to include the enzymatic process (e.g. cellular or acellular, e.g. a lysate or purified polypeptide mixture) by which ethanol is produced from a complex sugar, in particular, as a primary product of fermentation. According to the present invention the source of complex sugars is preferably lignocellulosic biomass.

The term "bioconversion" is intended to include the conversion of organic materials, such as plant or animal waste, into usable products or energy sources by biological processes or agents, such as certain microorganisms or enzymes.

The term "primary fermentation product" is intended to include non-gaseous products of fermentation that comprise greater than about 40%, 50%, 60%, 70%, 80%, 85%, 90%, 91%, 92%, 93%, 94%, 95% of total non-gaseous product. The primary fermentation product is the most abundant non-gaseous product. In certain embodiments of the invention, the primary fermentation product is ethanol.

The term "minor fermentation product" as used herein is intended to include non-gaseous products of fermentation that comprise less than 40%, for example 20%, 30%, 40%, of total non-gaseous product.

A first aspect of the invention relates to obtaining an increase in the yield of production of solvents, fuels and/or chemical intermediates by bacteria, more particularly by introducing, in said bacteria one or more nucleic acids encoding one or more cell wall degrading enzymes (and/or a scaffoldin capable of binding one or more cell wall degrading enzymes) such as to obtain better use of the lignocellulosic su bstrate.

According to this aspect, the present invention provides recombinant Gram-positive *Clostridia.* host cells comprising at least one nucleic acid encoding a plant cell wall degrading enzyme or a scaffoldin protein capable of binding one or more cell wall degrading enzymes, wherein said host cell is capable of expressing said nucleic acid and of producing and secreting said plant cell wall degrading enzyme or cellulosomal scaffoldin proteins.

In more particular embodiments, the recombinant Gram-positive *Clostridia* host cell of the present invention is a *Clostridium* species selected from the group comprising *Clostridium acetobutylicum, C. beijerinckii, C. saccharoperbutylacetonicum* or *C. saccharobutylicum,* preferably *C. acetobutylicum or C. beijerinckii* and more preferably *Clostridium acetobutylicum.*

The term cell wall degrading enzyme as used herein refers to either the complete enzyme or a functional fragment or variant thereof.

A "functional fragment" of an enzyme as used herein is a portion of the enzyme which retains the desired catalytic activity of the enzyme. For example, where the enzyme is cellulase or xylanase, a "functional fragment" is any fragment which respectively retains cellulase or xylanase activity. A "functional variant" of an enzyme as used herein has one or more substitutions such that the secondary conformation thereof remains unchanged but an activity of the enzyme is retained.

In particular embodiments the cell wall degrading enzymes are chosen from the group comprising acidic proteases, xylanases, cellulases, hemicellulase, arabinofuranosidases, lipolytic enzymes, pentosanases, fructanases, arabinases, mannosidases, pectinases, oxidoreductases, esterases, laccases, peroxidises and aryl alcool oxidases. More preferably, the one or more plant cell wall degrading enzymes are selected from the group consisting of cellulases, hemicellulases and lipolytic enzymes.

In particular embodiments, the one or more plant cell wall degrading enzymes are cellulases.

' A "cellulase" or "cellulase enzyme" according to the present invention is an enzyme that catalyzes the cellulolysis or hydrolysis of cellulose and includes, but is not limited to, endoglucanases, exoglucanases, endo-processive cellulases, cellobiohydrolases, cellobiases, oxidative cellulases, glucosidases, cellulose phosphorylases and/or other cellulases known in the art.

In further particular embodiments, the one or more plant cell wall degrading enzymes are hemicellulases.

The term "hemicellulase" or "hemicellulase enzyme" according to the present invention refers to an enzyme that catalyzes the hydrolysis of hemicellulose and includes, but is not limited to xylanases, ligninases, mannanases, and/or galactosidases.

Other plant cell wall degrading enzymes which may be used in the present invention include but are not limited to laccases, manganese peroxidase, lignin peroxidase, versatile peroxidase, or accessory enzymes such as cellobiose dehydrogenases, and aryl alcohol oxidases, cinnamoyl ester hydrolases able to release cinnamic acids such as ferulic acid and to hydrolyse diferulic acid cross-links between hemicellulose chains, such as feruloyl esterases, cinnamoyl esterases, and chlorogenic acid hydrolases.

In a particular embodiment of the present invention the one or more plant cell wall degrading enzymes are endoglucanases, exoglucanases and/or endo-processive cellulases.

The term "endoglucanases" as used herein refers to cellulases falling under the Enzyme Classification heading EC 3.2.1.4, also called β-1,4-endoglucanases, which cleave β-1,4-glycosidic linkages randomly along the cellulose chain. With exoglucanases are meant enzymes falling under the Enzyme Classification heading EC 3.2.1.91, also called cellobiohydrolases, which sequentially release cellobiose or glucose from one extremity of the cellulose chain. With endo-processive cellulases are meant enzymes falling under the Enzyme Classification heading EC 3.2.1.4/EC 3.2.1.91, which display a mixed mode of action with both endo- and exoglucanase activity.

In particular embodiments of the present invention the one or more plant cell wall degrading enzymes are chosen from the group comprising cellulase enzymes of *C. cellulolyticum,* and preferably selected from glycoside hydrolase family-5, 8, 9 and 48 cellulases.

In further particular embodiments of the present invention the one or more plant cell wall degrading enzymes are chosen from the group comprising *C. cellulolyticum* cellulase enzymes Cel48F, Cel9G, Cel9R, Cel9P, Cel9E, Cel9H, Cel9J, Cel9M, Cel8C, Cel44O, Cel5N and Cel5A, and functional fragments and/or functional variants of any of said cellulases.

In yet further particular embodiments of the present invention the plant cell wall degrading enzymes are chosen from the group comprising *Saccharophagus degradans* cellulase enzymes Cel5A, Cel5B, Cel5C, Cel5D, Cel5E, Cel5F, Cel5G, Cel5H, Cel5I, Cel6A, Cel5J, Cel9A, Cel9B, and functional fragments and/or functional variants of any one of said cellulases. The protein and nucleic acid accession numbers of particular embodiments of some of the above mentioned cellulases are given in Table 1.

**Table 1. Overview examples of cellulases which can be used in the present invention, their nucleic acid and protein accession numbers.**

| **Cellulase** | **Nucleotide accession number** | **Protein accession number** |
|---|---|---|
| From *Clostridium cellulolyticum* | | |
| Cel48F | U30321 | AAB41452 |
| Cel9G | M87018 | AAA73868 |
| Cel9R | - | ZP_01576699 |
| Cel9P | DQ778334 | ABG76972 |
| Cel9E | M87018 | AAA73869 |
| Cel9H | AF316823 | AAG45157 |
| Cel9J | AF316823 | AAG45158 |
| Cel9M | AF316823 | AAG45160 |
| Cel8C | M87018 | AAA73867 |
| Cel44O | - | ZP 01575597 |
| Cel5N | AF316823 | AAG45162 |
| Cel5A | M93096 | AAA51444 |

| From *Saccharophagus degradans* | | |
|---|---|---|
| Cel5A | - | YP_528472 or ADB28860 |
| Cel5B | - | YP_527962 or ABD81750 |
| Cel5C | - | YP_525801 or ABD79589 |
| Cel5D | - | YP_528108 or ABD81896 |
| Cel5E | - | YP_528398 or ABD82186 |
| Cel5F | - | YP_527046 or ABD80834 |
| Cel5G | - | YP_528708 or ABD82496 |
| Cel5H | - | YP_528706 or ABD82494 |
| Cel5J | - | YP_528887 or ABD82675 |
| Cel5J | - | YP_527966 or ABD81754 |
| Cel6A | - | YP_527744 or ABD81532 |
| Cel9A | - | YP_526110 or ABD79898 |
| Cel9B | - | YP_526123 or ABD79911 |

In particular embodiments the one or more plant cell wall degrading enzymes of the present invention are secreted. In this context the term "secretion" refers to the extracellular delivery of a polypeptide of interest, i.e. delivery outside a host cell. In particular this means that the polypeptide of interest is released in or accumulates outside a host cell, and for instance in the "environment" wherein said host cell is grown or is present.

By providing the plant cell wall degrading enzymes in a free form is meant that the enzymes are provided in an uncomplexed form, and are not attached or associated with other enzymes.

In particular embodiments, host cells according to the invention are capable of generating plant cell wall degrading enzymes which are provided in the form of a cellulolytic complex mimicking cellulosomes.

"Cellulosomes" are extracellular multi-enzymatic complexes that contain multiple enzymes required to break down carbohydrates. In particular, cellulosomes are composed of a scaffolding protein, which is attached to various cellulases, hemicellulases, and pectinases, that work synergistically to degrade complex cell-wall molecules. Such complexes allow a very efficient degradation of plant cell walls. The scaffolding proteins bring together the various other proteins in a signalling pathway and allow for their interaction. Cellulosomes are naturally encountered in some cellulolytic microorganisms.

The term "cellulosomal scaffolding protein" or "cellulosomal scaffoldin" as used herein is intended to refer to a scaffolding protein comprising one or more cohesins capable of anchoring one or more dockerins each of which is linked to a cell wall degrading enzyme thereby generating a cellulosome or cellulolytic complex. A cohesin is module of approx. 150 aminoacids (usually found in several copies in bacterial scaffoldins) which binds to its complementary module called the dockerin module. The dockerin module present on the cellulosomal catalytic sub-units of a cellulosome is usually composed of two conserved segments of 22 aminoacids connected by a linker. The cohesin/dockerin interaction is responsible for the attachment of the plant cell wall degrading enzymes to the scaffoldin. This interaction is in general calcium dependent and of strong affinity.

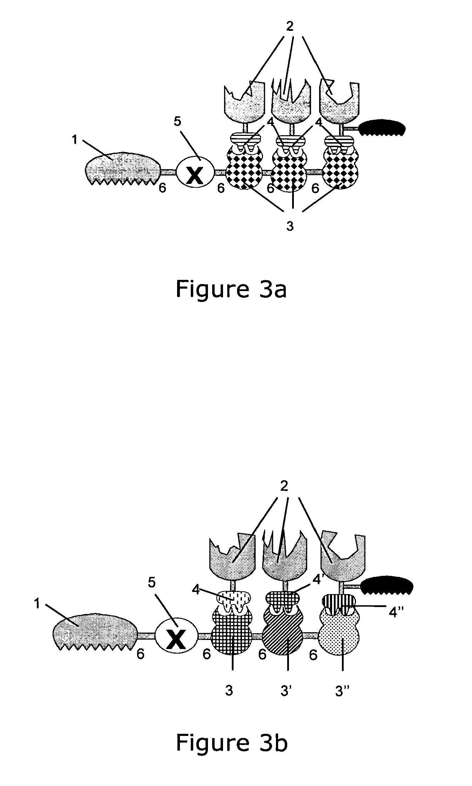

The term "cellulolytic complex" or "minicellulosome" as used herein is intended to refer to a recombinant cellulosome complex, of limited size compared to natural cellulosomes. They are characterized by a scaffolding protein containing a reduced number of cohesins (1-5) which will anchor a limited number of plant cell wall degrading enzymes appended with the appropriate dockerin modules. Minicellulosomes are shown in Figure 3A.

In particular embodiments the host cells according to the invention capable of generating plant cell wall degrading enzymes are provided with one or more nucleic acids encoding a cellulolytic complex. In further particular embodiments the host cells are provided with one or more nucleic acids encoding scaffoldin proteins, capable of binding one or more plant cell wall degrading enzymes so as to generate a functional cellulolytic complex.

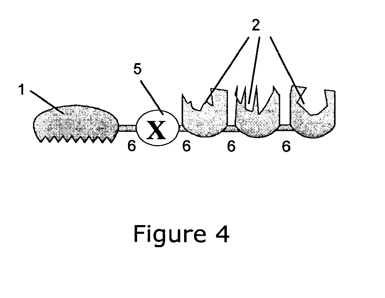

The cellulosomes or cellulolytic complexes envisaged in the context of the present invention may be hybrid and/or covalent. Hybrid cellulosomes are shown in Figure 3B. They are composed of a hybrid scaffoldin that contains a carbohydrate binding module and divergent and specific cohesins usually originating from different bacterial species, which attach to plant cell wall degrading enzymes engineered to bear the cognate complementary dockerin modules. The use of divergent and specific cohesin/dockerin docking systems to build hybrid cellulosomes allows to control the position of each catalytic sub-unit onto the hybrid scaffoldin. Covalent cellulosomes or cellulolytic complexes, are shown in Figure 4. These generally comprise a single protein containing the essential domains of a cellulosome, i.e. a cellulose binding module (such as a family 3a cellulose binding module), one or several "X" or "hydrophilic" modules and a limited number of catalytic modules. More particularly naturally occurring covalent cellulosomes tend to contain only two complementary catalytic modules, such as a family- 48 and 9 catalytic module and an accessory domain such as family 3c cellulose binding module.

The terms "X" or "hydrophilic" when referring to a domain or module as used herein accordingly refer to a hydrophilic domain of a cellulosomal scaffolding protein. Preferably in the context of the present invention hydrophilic modules of bacterial origin are envisaged, preferably from a bacteria of the genus *Clostridia,* e.g. from *Clostridium thermocellum, Clostridium cellulolyticum, Clostridium acetobutylicum, Clostridium josui* or *Clostridium cellulovorans*.

In particular embodiments of the invention, the recombinant host cells produce one or more plant cell wall degrading enzymes in the form of a cellulolytic complex wherein one or more co-expressed polypeptides, enzymes or cellulases as taught above, are comprised in a hybrid and/or covalent cellulosome (or cellulolytic complex) or minicellulosome. Such cellulosomes or minicellulosomes can provide for supra-molecular organisation of inter alia cellulose-binding and cellulose-depolymerising activities, thereby achieving greater efficiency of cellulose metabolism.

In further particular embodiments, the catalytic action of the one or more co-expressed polypeptides, enzymes or cellulases comprised in a cellulosome (cellulolytic complex) or minicellulosome are additive or complementary, preferably complementary, to the enzymatic activity of another cellulase. By means of example and not limitation, a first cellulase may be considered as having activity complementary to a second cellulase if the first and second cellulases act preferentially on distinct substrates (such as, e.g., crystalline cellulose, semicrystalline cellulose, amorphous cellulose or hemicellulose), or if the first and second cellulases produce distinct products (e.g., distinct populations of sugar monomers and/or oligomers), or if the first cellulase acts preferentially on reaction products of the second cellulase or vice versa, etc.

In particular embodiments of the present invention the cellulolytic complex is a hybrid complex which is composed of a scaffoldin protein preferably selected from the group CipC of *C. cellulolyticum,* CipA of *C. thermocellum,* CbpA of *C. cellulovorans,* CipA of *C. acetobutylicum* and CipA of *C. josui,* and at least one, preferably at least two and more preferably 2, 3, 4, 5, 6, 7 or 8 of the above mentioned cellulases and functional fragments and/or functional variants of any of said cellulases appended to said scaffoldin protein with appropriate dockerins-cohesin domains. Figure 3 for instance illustrates an embodiment of a hybrid cellulolytic complex comprising a scaffoldin protein (1), cellulases (2), cohesins (3), dockerins (4), a hydrophilic domain (5) and linkers (6).

In further particular embodiments, the host cells of the invention produce and secrete a two-component minicellulosome composed of a miniscaffoldine (CBM-X2-Coh) and one mannanase (Man5K), such as described by Mingardon et al. (2005, Appl. Environ. Microbiol. 71: 1215-1222).

In further particular embodiments, the host cells according to the present invention comprise a sequence which encodes a cellulosomal scaffolding protein such as those described above, preferably selected from the group CipC of *C. cellulolyticum,* CipA of *C. thermocellum,* CbpA of *C. cellulovorans,* CipA of *C. acetobutylicum* and CipA of *C. josui,* capable of binding at least one, preferably at least two and more preferably 2, 3, 4, 5, 6, 7 or 8 of the above mentioned cellulases and functional fragments and/or functional variants of any of said cellulases through interaction of appropriate dockerin/cohesin domains.

In certain embodiments of the present invention the cellulolytic complex is a covalent complex which is composed of a scaffoldin protein preferably selected from the group CipC of *C. cellulolyticum,* CipA of *C. thermocellum,* CbpA of *C. cellulovorans,* CipA of *C. acetobutylicum* and CipA of *C. josui,* and at least one, preferably at least two and more preferably 2, 3, 4, 5, 6, 7 or 8 of the above mentioned cellulases and functional fragments and/or functional variants of any of said cellulases which are operably linked to said scaffoldin protein. Figure 4 for instance illustrates an embodiment of a covalent cellulolytic complex comprising a scaffoldin protein (1), cellulases (2), a hydrophilic domain (5) and linkers (6).

Another aspect of the present invention provides *Clostridia* host cells comprising a recombinant solventogenic metabolism which is adapted to increase the production of solvents, fuels and/or chemical intermediates. More preferably a host cell according to this aspect of the invention comprises a recombinant solventogenic metabolism adapted to increase ethanol production.

The metabolism of host cells of the present invention produces besides ethanol as a primary fermentation product also lactate, acetate, acetone, butanol, isopropanol, propanol, 1,2 propanediol and butyrate as minor fermentation products. The glycolysis metabolism converts complex sugars into pyruvate whereas pyruvate can subsequently be used as the source for the primary fermentation product and the minor fermentation products.

According to particular embodiments, *Clostridia* host cells are provided wherein the recombinant solventogenic metabolism comprises at least one nucleic acid encoding an enzyme that converts pyruvate to acetaldehyde optionally combined with at least one heterologous nucleic acid encoding an enzyme that converts acetaldehyde to ethanol wherein said host cell is capable of expressing said nucleic acid(s). Preferably the at least one nucleic acid encoding an enzyme that converts pyruvate to acetaldehyde is a heterologous nucleic acid whereas the at least one nucleic acid encoding an enzyme that converts acetaldehyde to ethanol can either be an endogenous or a heterologous nucleic acid. In particular embodiments, host cells are provided which comprise at least one heterologous nucleic acid encoding an enzyme that converts acetaldehyde to ethanol wherein said host cell is capable of expressing said at least one nucleic acid.

Particular embodiments of the host cells, and preferably *C. acetobutylicum,* comprising recombinant solventogenic metabolism of the present invention, comprise at least one nucleic acid encoding an enzyme that converts pyruvate to acetaldehyde, whereas the enzyme converting acetaldehyde to ethanol is encoded by an endogenous nucleic acid naturally occurring in the host cell.

Further particular embodiments of the host cell comprising recombinant solventogenic metabolism of the present invention comprise at least one nucleic acid encoding an enzyme that converts pyruvate to acetaldehyde and at least one heterologous nucleic acid encoding an enzyme that converts acetaldehyde to ethanol. The heterologous nucleic acid encoding an enzyme that converts acetaldehyde to ethanol provides an additional source of enzymes, supporting or replacing the naturally occurring enzymes for the conversion of acetaldehyde to ethanol.

The recombinant and/or heterologous polynucleotide sequences used in the context of the present invention are involved in at least one step in the bioconversion of a lignocellulose source to ethanol or another solvent, fuel or chemical intermediate of interest. Accordingly, these polynucleotide sequences include genes encoding a polypeptide such as an *alcohol dehydrogenase (adh), a pyruvate decarboxylase (pdc),* (a) secretory protein/s, lignocellulose degrading enzymes and/or other polypeptides involved in at least one step in the bioconversion of a lignocellulose source to a solvent, fuel or chemical intermediate such as ethanol.

In particular embodiments, the nucleic acid encoding an enzyme that converts pyruvate to acetaldehyde is pyruvate decarboxylase (*pdc*).

Pyruvate decarboxylase (*pdc*) nucleic acid encodes for a pyruvate decarboxylase (PDC), an enzyme catalysing the decarboxylation of pyruvic acid to acetaldehyde and carbon dioxide. A *pdc* nucleic acid may be obtained from any source known in the art such as corn, yeast or bacteria and preferably from *Zymomonas mobilis.* More preferably a *pdc* nucleic acid which may be used in the present invention is from *Zymomonas mobilis* subsp *mobilis* ZM4 (with gene locus tag ZMO1360). Alternatively other *pdc* nucleic acids which may be used in the present invention may be selected from the group comprising PDC1 (with gene locus tag YLR044C), PDC2 (with gene locus tag YDR081C), PDC5 (with gene locus tag YLR134W), PDC6 (with gene locus tag YGR087C) from *Saccharomyces cerevisiae, pdc* nucleic acid from *Zymobacter palmae* (with gene accession number AF474145), *pdc* nucleic acid from *Acetobacter pasteurianus* (with gene accession number AF368435), *pdc* nucleic acid from *Sarcina ventriculi* (with gene accession number AF354297).

By providing a host cell, and preferably *C. acetobutylicum* with a *pdc* nucleic acid encoding a pyruvate decarboxylase enzyme, the conversion of pyruvate into acetaldehyde is increased thereby driving the solventogenic metabolism of said host cell toward the production of ethanol (or another solvent, fuel or chemical intermediate of interest).

In a preferred embodiment a host cell comprises one or more pyruvate decarboxylase (*pdc*) nucleic acids.

In particular embodiments, host cells, and preferably *C. acetobutylicum,* comprising recombinant solventogenic metabolism according to the present invention with one or more *pdc* nucleic acids encoding a pyruvate decarboxylase enzyme are characterized by an increase in the production of one or more solvents, fuels or chemical intermediates of interest, such as ethanol. More particularly the increase is at least a 1.5 fold increase, preferably at least a 2 fold increase, more preferably at least a 2.5 fold increase and more preferably at least a 3 fold increase compared to the organism not comprising the recombinant solventogenic metabolism according to the invention, and/or, where appropriate, compared to the ethanol yield from glucose in a typical ABE fermentation. Typically the ethanol yield from glucose in ABE fermentation ranges between 0.1 and 0.15 mol ethanol per mol glucose. The host cell of the present invention, and preferably *C. acetobutylicum* with one or more *pdc* nucleic acids encoding a pyruvate decarboxylase enzyme have shown an ethanol production ranging between 0.15 and 0.5 mol ethanol per mol glucose, preferably between 0.2 and 0.4 mol ethanol per mol glucose and more preferably between 0.25 and 0.35 mol ethanol per mol glucose.

In a preferred embodiment the pyruvate decarboxylase nucleic acid is obtained from *Zymomonas mobilis.*

In the host cells of the present invention the nucleic acid encoding an enzyme that converts pyruvate to acetaldehyde works together with an enzyme that converts acetaldehyde to ethanol to ensure optimal ethanol production. In particular embodiments the enzyme that converts acetaldehyde to ethanol is alcohol dehydrogenase.

An alcohol dehydrogenase is an enzyme catalysing the interconversion between aldehydes or ketones and alcohols with the oxidation of NADH to NAD⁺. More preferably, the alcohol dehydrogenase catalyzes the conversion of acetaldehyde to ethanol thereby oxidizing NADH to NAD⁺. The nucleic acid encoding an alcohol dehydrogenase (*adh)* present in the host cells according to the invention can either be an endogenous or a heterologous nucleic acid. The heterologous *adh* nucleic acid can be obtained from any source known in the art such as horse, yeast, human, insect or bacteria and preferably from *Saccharomyces cerevisiae.*

More preferably a heterologous adh nucleic acid which may be used in the present invention is ADH1 from *Saccharomyces cerevisiae* strain S288C (with gene locus tag YOL086C). Additionally or alternatively, other *adh* nucleic acids which may be used in the present invention may be selected from the group comprising ADH2 (with gene locus tag YMR303C), ADH3 (with gene locus tag YMR083W), ADH4 (with gene locus tag YGL256W), ADH5 (with gene locus tag YBR145W and YDL168W), ADH6 (with gene locus tag YMR318C) and ADH7 (with gene locus tag YCR105W) from *Saccharomyces cerevisiae* or ADHB (with gene locus tag ZMO159) from *Zymomonas mobilis,* or other ADH (with gene locus tags ZMO0090, ZMO0797, ZMO1222, ZMO1236, ZMO1372, ZMO1561, ZMO1576, ZMO1578, ZMO1946) from *Zymomonas mobilis,* or ADHC (with gene locus tag P25437), ADHE (with gene locus tag P17547), ADHP (with gene locus tag P39451) from *E. coli.*

In a preferred embodiment the alcohol dehydrogenase nucleic acid is obtained from *Saccharomyces cerevisiae.*

In another embodiment of the present invention the nucleic acids are plasmid borne and more preferably the *pdc* and the heterologous adh nucleic acids are plasmid borne.

In yet another embodiment of the present invention the nucleic acids are incorporated into the chromosome of said host cell.

By providing a host cell, and preferably *C. acetoburylicum,* comprising recombinant solventogenic metabolism according to this aspect of the present invention, with a heterologous *adh* nucleic acid encoding an alcohol dehydrogenase enzyme, the conversion of acetaldehyde to ethanol is enhanced when the endogenous alcohol dehydrogenase activity is limiting ethanol production thereby driving further the solventogenic metabolism of a host cell toward the production of ethanol.

Accordingly, by providing a host cell and preferably *C. acetobutylicum* comprising recombinant solventogenic metabolism according to this aspect of the present invention, with heterologous adh nucleic acids encoding for alcohol dehydrogenase enzymes the production of one or more solvents, fuels or chemical intermediates of interest, such as ethanol is increased.

Preferably, the production of the solvent, fuel and/or chemical intermediate is increased with at least 10%, 20%, 25%, 30%, 40%, 50%, 75%, 100% compared to the production by a comparable host cell not comprising the recombinant metabolism according to the invention and/or, where appropriate, compared to the ethanol yield of a typical ABE fermentation. In particular embodiments, a host cell of the present invention provides at least a 2 fold, preferably at least a 3 fold, more preferably at least a 4 fold and more preferably at least a 5 fold increase in the ethanol production compared to the ethanol yield from glucose in a typical ABE fermentation.

Accordingly, the present invention provides cultures of the recombinant host cells of the present invention, more particularly cultures of *C. acetobutylicum,* wherein the yield of solvent, fuel or chemical intermediate of interest, such as ethanol is increased with at least 10%, 20%, 25%, 30%, 40%, 50%, 75%, 100% compared to the yield of said solvent, fuel or chemical intermediate from a culture of host cells not comprising the recombinant metabolism of the present invention and/or where appropriate compared to the ethanol yield of a typical ABE fermentation. Particular embodiments of the invention provide cultures of host cells demonstrating an ethanol production ranging between 0.15 and 0.5 mol ethanol per mol glucose, preferably between 0.2 and 0.4 mol ethanol per mol glucose and more preferably between 0.25 and 0.35 mol ethanol per mol glucose.

Further particular embodiments of the invention provide recombinant host cells and preferably *C. acetobutylicum* comprising a recombinant solventogenic metabolism as described above and further comprising at least one nucleic acid encoding a plant cell wall degrading enzyme, wherein the host cell is capable of expressing said nucleic acid and of producing and secreting the plant cell wall degrading enzyme as described hereinabove.

Yet another aspect of the present invention provides recombinant host cells which comprise a recombinant solventogenic metabolism comprising a mutation in at least one nucleic acid encoding for an enzyme in a metabolic pathway in said host cell, wherein the pathway produces a metabolite other than acetaldehyde from pyruvate or ethanol from acetaldehyde (or other than any metabolite which is relevant in the production of the solvent, fuel or chemical intermediate of interest by the host cell), and wherein the mutation results in a reduced production of the metabolite.

In particular embodiments, host cells are provided comprising a recombinant solventogenic metabolism as described hereinabove, i.e. comprising at least one nucleic acid encoding an enzyme that converts pyruvate to acetaldehyde in association or not with at least one heterologous nucleic acid encoding an enzyme that converts acetaldehyde to ethanol wherein said host cell is capable of expressing said nucleic acid(s) and further comprising a mutation in at least one nucleic acid encoding for an enzyme in a metabolic pathway in said host cell, wherein said pathway produces a metabolite other than acetaldehyde from pyruvate or ethanol from acetaldehyde, and wherein said mutation results in a reduced production of said metabolite.

In particular embodiments of this aspect of the present invention the mutation is a deletion, insertion and/or base change mutation.

In further particular embodiments of this aspect of the present invention the mutation is a mutation in at least one nucleic acid encoding an enzyme chosen from the group comprising pyruvate ferredoxin oxidoreductase (*pfor*)*,* phosphotransacetylase (*pta*), acetate kinase (*ak*), coenzym A transferase (*ctfAB*)*,* acetoacetate decarboxylase (*adc*), phosphotransbutyrylase (*ptb*), butyrate kinase (*bk*)*,* lactate dehydrogenase (*Idh*)*,* thiolase (*thl*)*,* β-hydroxybutyryl coenzyme A dehydrogenase (*hbd*), crotonase (*crt*), butyryl coenzyme A dehydrogenase *(bcd*)*,* bifunctional butyraldehyde-butanol dehydrogenase (*aad*/*adhE, adhE2)* and/or butanol dehydrogenase (*bdhAB*) and preferably chosen from the group consisting of pyruvate ferredoxin oxidoreductase (*pfor*)*,* phosphotransacetylase (*pta*)*,* acetate kinase (*ak*)*,* coenzym A transferase (*ctfAB*)*,* acetoacetate decarboxylase (*adc*), phosphotransbutyrylase (*ptb*)*,* butyrate kinase (*bk*)*,* lactate dehydrogenase (*Idh*)*,* bifunctional butyraldehyde-butanol dehydrogenase (*aadladhE, adhE2*) and/or butanol dehydrogenase *(bdhAB*).

According to particular embodiments of the present invention the mutation is a mutation in at least one nucleic acid encoding an enzyme in the metabolic pathway that converts pyruvate to lactate and preferably in the lactate dehydrogenase (*Idh*) nucleic acid.

The lactate dehydrogenase (*Idh*) nucleic acid encodes for a lactate dehydrogenase (LDH), an enzyme catalysing the interconversion of pyruvate and lactate with concomitant interconversion of NADH and NAD+. A mutation in the *Idh* nucleic acid would disturb the production from the minor fermentation product lactate. This leads to the direction of the metabolic pathway towards the other solvents, fuels and/or chemical intermediates and a more efficient production of these solvents, fuels and/or chemical intermediates, and preferably ethanol. A particular embodiment of a lactate dehydrogenase (*Idh*) enzyme is encoded by the CAC0267 gene in *C. acetobutylicum* ATCC 824 genome sequence (Nölling et al., 2001, J. Bacteriol. 183:4823-4838).

According to particular embodiments of the present invention the mutation is a mutation in at least one nucleic acid encoding an enzyme in the metabolic pathway that converts pyruvate to acetyl coenzyme A and preferably in the pyruvate ferredoxin oxidoreductase (*pfor*) nucleic acid.

The pyruvate ferredoxin oxidoreductase (*pfor*) nucleic acid encodes for a pyruvate ferredoxin oxidoreductase (PFOR), an enzyme catalysing the oxidative decarboxylation of pyruvate to acetyl coenzyme A and CO₂. Acetyl coenzyme A is a precursor for the solventogenic metabolic pathways and acetyl coenzyme A can further be used for the production of ethanol, acetate, acetone, butyrate and butanol. By providing a mutation in the *pfor* nucleic acid the production of acetyl coenzyme A from pyruvate would be reduced or inhibited, hence rendering the ethanol production more efficient. Since a host cell of the present invention is provided with a *pdc* nucleic acid which converts pyruvate to acetaldehyde, the production of ethanol from pyruvate is more efficient since all pyruvate is converted to acetaldehyde, and because of the mutation in the *pfor* nucleic acid pyruvate is no longer or in a lesser amount converted into acetyl coenzyme A.

Sequence analysis of the *C. acetobutylicum* ATCC 824 genome (Nölling et al., 2001, J. Bacteriol. 183:4823-4838) showed two open reading frames CAC2229 and CAC2499 encoding putative pyruvate ferredoxin oxidoreductase: they exhibit high amino acid sequence homology and identity with the pyruvate ferredoxin oxidoreductase of *Desulfovibrio africanus* (CAC2229: identity 54%, homology 67% and CAC2499: identity 53%, homology 66%).

According to further particular embodiments of the present invention the mutation is a mutation in at least one nucleic acid encoding an enzyme in the metabolic pathway that converts acetyl coenzyme A into acetate. This conversion requires a phosphotransacetylase (*pta*) and an acetate kinase (*ak*)*.* In particular embodiments these enzymes are encoded respectively by CAC1742 and CAC1743 in *C. acetobutylicum* ATCC 824 genome sequence (Nölling et al., 2001, J. Bacteriol. 183:4823-4838).

According to further particular embodiment of the present invention the mutation is a mutation in at least one nucleic acid encoding an enzyme in the metabolic pathway that converts acetyl coenzyme A into butyryl coenzyme A. This conversion requires an acetyl-coenzyme A acetyltransferase or thiolase (*thlA*)*,* a β-hydroxybutyryl coenzyme A dehydrogenase (*hbd*)*,* a crotonase (crt), a butyryl coenzyme A dehydrogenase (*bcd*)*.* In particular embodiments, these enzymes are encoded respectively by CAC2873, CAC2708, CAC2712 and CAC2711 nucleic acids in *C. acetobutylicum* ATCC 824 genome sequence (Nölling et al., 2001, J. Bacteriol. 183:4823-4838).

According to further particular embodiments of the present invention the mutation is a mutation in at least one nucleic acid encoding an enzyme in the metabolic pathway that converts acetoacetyl coenzyme A into acetone. This conversion requires a coenzyme A transferase (*ctfAB*) and an acetoacetate decarboxylase (*adc*). In particular embodiments these enzymes are encoded respectively by CAP0163-CAP0164 and CAP165 in *C. acetobutylicum* ATCC 824 genome sequence (Nölling et al., 2001, J. Bacteriol. 183:4823-4838).

According to further particular embodiments of the present invention the mutation is a mutation in at least one nucleic acid encoding an enzyme in the metabolic pathway that converts butyryl coenzyme A into butyrate. This conversion requires a phosphotransbutyrylase (*ptb*) and a butyrate kinase (*bk*). In particular embodiments these enzymes are encoded respectively by CAC3076 and CAC3075 in *C. acetobutylicum* ATCC 824 genome sequence (Nölling et al., 2001, J. Bacteriol. 183:4823-4838).

According to further particular embodiment of the present invention the mutation is a mutation in at least one nucleic acid encoding an enzyme in the metabolic pathway that converts butyryl coenzyme A into butanol. This conversion requires a bifunctional butyraldehyde-butanol dehydrogenase (*aad*/*adhE, adhE2*) and a butanol dehydrogenase (*bdhAB*). In particular embodiments these enzymes are encoded respectively by CAP0162, CAP0035, CAC3298 and CAC3299 in *C. acetobutylicum* ATCC 824 genome sequence (Nölling et al., 2001, J. Bacteriol. 183:4823-4838).

Further mutations in enzymes which ensure the production of "undesirable" metabolites other than metabolites which are relevant in the production of solvents, fuels and/or chemical intermediates by the host cells of interest, and which have as a result the decreased production of these "undesirable" metabolites, can be envisaged by the skilled person.

The present state of the art provides a wide variety of techniques that can be used for the inactivation, deletion or replacement of genes. Molecular techniques for genetic manipulations in *Clostridia* include, but are not limited to:
(i) electrotransformation techniques and shuttle vectors for heterologous overexpression in *C. acetobutylicum* (Mermelstein et al., 1992, Biotechnology, 10:190-195) and in *C. beijerinckii* (Birrer et al., 1994, Appl Microbiol Biotechnol., 41:32-48),
(ii) techniques for single gene mutation such as gene inactivation by single crossing over with non-replicative plasmid (Green et al., 1996, Microbiology, 142:2079-2086) and gene inactivation with a replicative plasmid capable of double-crossover chromosomal integration (Harris et al., 2002, J. Bacteriol., 184:3586-3597) mutagenesis system based on mobile intron : ClosTtron system (Heap et al., J. Microbiological Methods, 2007, 70:452-464),
(iii)techniques for multiple unmarked mutations in the same strain such as:
   (A) the technique described in PCT/EP2006/066997, based on (a) deletion and replacement of the target gene by an antibiotic resistance gene by a double crossover integration through homologous recombination of a replicative integrative plasmid, giving segregationally highly stable mutants; (b) removing of the antibiotic resistance gene with the Flp recombinase system from Saccharomyces cerevisiae allowing the repeated use of the method for construction of multiple, unmarked mutations in the same strain and (c) a *C. acetobutylicum* strain deleted for the upp gene, encoding uracil phosphoribosyl transferase, thus allowing the use of 5-fluorouracyl as a counter selectable marker and a positive selection of the double crossover integrants, or
   (B) the use of "second-generation" ClosTron plasmids in which the ermB gene is flanked by FRT sites allowing the removal of the ermB gene by expression of FLP recombinase (Heap et al., 24-27th february 2008, Non-pathogenic Clostridia, a Marie Curie conference, Toulouse, France),
and/or a combination of any of the techniques described above.

In specific embodiments of the invention methods are provided wherein a microorganism is modified to be unable to convert pyruvate to acetyl coenzyme A as a result of the deletion of or the inactivation of the polynucleotide encoding for pyruvate ferredoxin oxidoreductase (*pfor*)*,* e.g. using the method recently described in patent application PCT/EP2006/066997. This strategy provides that the production of acetyl coenzyme A from pyruvate is reduced or inhibited and the production of ethanol is increased and more efficient.

According to particular embodiments of the present invention the mutation provided in the recombinant solventogenic metabolism of the host cells of the invention is a mutation in at least one nucleic acid encoding an enzyme in the metabolic pathway that converts acetyl coenzyme A to acetate and preferably a mutation in at least one nucleic acid encoding an enzyme chosen from the group comprising phosphotransacetylase (*pta*) and acetate kinase (*ak*)*.*

For the conversion of acetyl coenzyme A to acetate, acetyl coenzyme A is converted to acetyl phosphate by a phosphotransacetylase (PTA), encoded by a phosphotransacetylase (*pta*) nucleic acid, and the acetyl phosphate is converted to acetate by acetate kinase (AK), encoded by a acetate kinase (*ak*) nucleic acid.

A mutation in the *pta* and/or *ak* nucleic acids will lead to a disturbance of the metabolic pathway for the production of acetate. This mutation leads to a decreased production of acetate and thereby an increased production of the other solvents, fuels and/or chemical intermediates and especially ethanol.

In other embodiments of the present invention the mutation is a mutation in at least one nucleic acid encoding an enzyme in the metabolic pathway that converts acetyl coenzyme A to butyryl coenzyme A and preferably a mutation in at least one nucleic acid encoding an enzyme chosen from the group comprising thiolase (*thl*)*,* β-hydroxybutyryl coenzyme A dehydrogenase (*hbd*), crotonase (*crt*), butyryl coenzyme A dehydrogenase (*bcd*)*.*

Acetyl coenzyme A is also converted to acetoacetyl coenzyme A by acetyl-coenzyme A acetyltransferase or thiolase (thl), encoded by a thiolase (*thl*) nucleic acid. The enzymes β-hydroxybutyryl coenzyme A dehydrogenase (*hbd*), crotonase (*crt*), butyryl coenzyme A dehydrogenase (*bcd*) further convert acetoacetyl coenzyme A to butyryl coenzyme A.

A mutation in the *thl, hbd, crt,* and/or *bcd* nucleic acids will lead to a disturbance of the metabolic pathway for the production of butanol and butyrate. This mutation leads to a decreased production of butanol and butyrate and thereby an increased production of the other solvents, fuels and/or chemical intermediates and especially ethanol.

According to an embodiment of the present invention a host cell is provided comprising a mutation in at least one nucleic acid encoding an enzyme in the metabolic pathway that converts acetoacetyl coenzyme A to acetone and preferably a mutation in at least one nucleic acid encoding an enzyme chosen from the group comprising coenzym A transferase (*ctfAB*) and acetoacetate decarboxylase (*adc*).

For the conversion of acetoacetyl coenzyme A to acetone, acetoacetyl coenzyme A is converted to acetoacetate by a coenzyme A transferase (*ctfAB*)*,* encoded by a coenzyme A transferase (*ctfAB*) nucleic acid, and the acetoacetate is converted to acetone by acetoacetate decarboxylase (ADC), encoded by a acetoacetate decarboxylase (*adc*) nucleic acid.

A mutation in the *ctfAB* and/or *adc* nucleic acids will lead to a disturbance of the metabolic pathway for the production of acetone. This mutation leads to a decreased production of acetone and thereby an increased production of the other solvents, fuels and/or chemical intermediates and especially ethanol.

According to an embodiment of the present invention the mutation is a mutation in at least one nucleic acid encoding an enzyme in the metabolic pathway that converts butyryl coenzyme A to butyrate and preferably a mutation in at least one nucleic acid encoding an enzyme chosen from the group comprising phosphotransbutyrylase (*ptb*) and butyrate kinase (*bk*)*.*

A mutation in the *ptb* and/or *bk* nucleic acids will lead to a disturbance of the metabolic pathway for the production of butyrate. This mutation leads to a decreased production of butyrate and thereby an increased production of the other solvents, fuels and/or chemical intermediates and especially ethanol.

According to yet an embodiment of the present invention the mutation is a mutation in at least one nucleic acid encoding an enzyme in the metabolic pathway that converts butyryl coenzyme A to butanol and preferably a mutation in at least one nucleic acid encoding an enzyme chosen from the group comprising bifunctional butyraldehyde-butanol dehydrogenase (*aad*/*adhE, adhE2*) and butanol dehydrogenase *(bdhAB).*

A mutation in the *aad*/*adhE, adhE2* and/or *bdhAB* nucleic acids will lead to a disturbance of the metabolic pathway for the production of butanol. This mutation leads to a decreased production of butanol.

A further aspect of the invention provides recombinant host cells comprising:
a mutation in at least one nucleic acid encoding for an enzyme in a metabolic pathway in said host cell, wherein said pathway produces a metabolite other than acetaldehyde from pyruvate or ethanol from acetaldehyde (or other than a metabolite which is relevant for the production of the solvent, fuel and/or chemical intermediate of interest), and wherein said mutation results in a reduced production of said metabolite as described hereinabove,
further comprise:
   at least one nucleic acid encoding an enzyme that converts pyruvate to acetaldehyde and at least one nucleic acid encoding an enzyme that converts acetaldehyde to ethanol;
   wherein the host cell is capable of expressing said nucleic acid according to an aspect of the invention described herein.

Organisms according to this aspect of the invention can be obtained, e.g., by introducing a mutation in at least one nucleic acid encoding for an enzyme in a metabolic pathway in said host cell, wherein said pathway produces a metabolite other than acetaldehyde from pyruvate or ethanol from acetaldehyde as described hereinabove and by further introducing into the host cell at least one nucleic acid encoding an enzyme that converts acetaldehyde to ethanol wherein said host cell is capable of expressing said nucleic acid according to an aspect of the invention described herein.

Optionally the mutation of at least one nucleic acid and introduction of at least one other nucleic acid are performed simultaneously, for instance by one of the methods described below:
1) the technique described in PCT/EP2006/066997, based on (a) deletion and replacement of the target gene by an antibiotic resistance gene by a double crossover integration through homologous recombination of a replicative integrative plasmid, giving segregationally highly stable mutants; (b) removing of the antibiotic resistance gene with the Flp recombinase system from Saccharomyces cerevisiae allowing the repeated use of the method for construction of multiple, unmarked mutations in the same strain and (c) a *C. acetobutylicum* strain deleted for the upp gene, encoding uracil phosphoribosyl transferase, thus allowing the use of 5-fluorouracyl as a counter selectable marker and a positive selection of the double crossover integrants, or
2) the use of "second-generation" ClosTron plasmids in which the ermB gene is flanked by FRT sites allowing the removal of the ermB gene by expression of FLP recombinase (Heap et al., 24-27th february 2008, Non-pathogenic Clostridia, a Marie Curie conference, Toulouse, France),

In particular embodiments of this aspect of the invention, host cells are provided wherein the mutation or the deletion or the inactivation of the polynucleotide encoding for pyruvate ferredoxin oxidoreductase (*pfor*) is combined with introducing into the host cell at least one nucleic acid encoding an enzyme that converts pyruvate to acetaldehyde and at least one nucleic acid encoding an enzyme that converts acetaldehyde to ethanol wherein said host cell is capable of expressing said nucleic acid according to an aspect of the invention described herein. In further particular embodiments, solventogenic microorganisms are provided wherein the replacement of the *pfor* polynucleotide is combined with the introduction of a polynucleotide encoding for *pdc* and *adh.*

Construction of strains according to the present invention in which the pyruvate ferredoxin oxidoreductase (*pfor*) genes are deleted and replaced by the synthetic *pdc-adh1* operon can be performed, as follows. Sequence analysis of the *C. acetobutylicum* ATCC 824 genome (Nölling et al., 2001, J. Bacteriol. 183:4823-4838) shows two open reading frames CAC2229 and CAC2499 encoding putative pyruvate ferredoxin oxidoreductase: they exhibit high amino acid sequence homology and identity with the pyruvate ferredoxin oxidoreductase of *Desulfovibrio africanus* (CAC2229: identity 54%, homology 67% and CAC2499: identity 53%, homology 66%). To delete CAC2229 or CAC2499 genes, the homologous recombination method described in patent application PCT/EP2006/066997 can be used. The strategy allows the insertion of an erythromycin resistance cassette, and of a *pdc-adhl* synthetic operon cassette while deleting the entire gene concerned.

The CAC2229 deletion cassette can be constructed by a procedure such as, but not limited to, the following. Two DNA fragments surrounding CAC2229 are PCR-amplified from *C. acetobutylicum* ATCC 824 genomic DNA. Primers can be used which introduce suitable restriction sites. DNA fragments can then be joined in a PCR fusion experiment. The resulting nucleotide fragment is then cloned in pCR4-Blunt-TOPO yielding pTOPO:CAC2229. At the unique NheI site of the pTOPO:CAC2229, a pdc-adh1 synthetic operon cassette can be cloned. The *pdc-adh1* cassette is PCR-amplified from *pSOS95-pdcadhl,* introducing the NheI site at both ends. At the unique StuI site of the pTOPO:CAC2229, an antibiotic resistance MLS gene with FRT sequences on both ends can be introduced from the StuI fragment of pUC18-FRT-MLS2.

The CAC2499 deletion cassette can be constructed as follows. Two DNA fragments surrounding CAC2499 are PCR-amplified from *C. acetobutylicum* ATCC 824 genomic DNA using suitable primers to introduce restriction sites. DNA fragments are then joined in a PCR fusion experiment with suitable primers. The resulting nucleotide fragment can be cloned in pCR4-Blunt-TOPO to yield pTOPO:CAC2499. At the unique NheI site of the pTOPO:CAC2499, the pdc-adh1 synthetic operon cassette is cloned. The *pdc-adhl* cassette can be PCR-amplified from *pSOS95-pdcadhl,* introducing the NheI site at both ends. At the unique StuI site of the pTOPO:CAC2499, an antibiotic resistance MLS gene with FRT sequences on both ends can be introduced from the StuI fragment of pUC18-FRT-MLS2.

The CAC2229 and CAC2499 deletion cassettes obtained after BglII digestion of the resulting plasmids can be cloned into pCons::upp at the BamHI site to yield the *pREP*Δ*CAC2229::upp* plasmid and the *pREP*Δ*CAC2499::upp* plasmid, respectively. Each plasmid can then be used to transform by electroporation *C. acetobutylicum* Δ*cac15*Δ*upp* strain. Clones resistant to erythromycin (40 µg ml⁻¹) are then selected on Petri dishes. One colony is then cultured for 24 hours in liquid synthetic medium with 40 µg ml⁻¹ erythromycin and 100 µl of undiluted culture is plated on RCA with 40 µg ml⁻¹ erythromycin and 100 µM 5-Fluorouracyl (5-FU). Colonies resistant to both erythromycin and 5-FU can then be replica plated on both RCA with 40 µg ml⁻¹ erythromycin and RCA with 50 µg ml⁻¹ thiamphenicol to select clones in which 5-FU resistance is also associated with thiamphenicol sensitivity. The genotype of the clones resistant to erythromycin and sensitive to thiamphenicol is checked by PCR analyses with the couples of primers located outside of the deletion cassettes, respectively. The two strains *C. acetobutylicum* Δ*cac15*Δ*upp*Δ*CAC2229::mlsR-pdc-adhl* and *C. acetobutylicum* Δ*cac15*Δ*upp*Δ*CAC2499:: mlsR -pdc-adh1* which have lost respectively the *pREP*Δ*CAC2229::upp* plasmid and the *pREP*Δ*CAC2499::upp* plasmid can then be isolated. The two strains *C. acetobutylicum* Δ*cac15*Δ*upp*Δ*CAC2229::mlsR-pdc-adhl* and *C. acetobutylicum* Δ*cac15*Δ*upp*Δ*CAC2499:: mlsR-pdc-adh1* can then be transformed with pCLFI.1 vector which expresses the Flp1 gene coding for the Flp recombinase of *S. cerevisiae.* After selection for resistance to 50 µg ml⁻¹ thiamphenicol on Petri dishes, one colony can be cultured on liquid synthetic medium with 50 µg ml⁻¹ thiamphenicol and appropriate dilutions are plated on RCA with 50 µg ml⁻¹ thiamphenicol. Thiamphenicol resistant clones are replica plated on both RCA with 40 µg ml⁻¹ erythromycin and RCA with 50 µg ml⁻¹ thiamphenicol. The genotype of the clones sensitive to erythromycin and resistant to thiamphenicol can be checked by PCR analyses. In order to lose pCLF1.1, two successive 24 hour cultures of *C*. *acetobutylicum* Δ*cac15*Δ*upp*Δ*CAC2229::pdc-adhl* and *C. acetobutylicum* Δ*cac15*Δ*upp*Δ*CAC2499::pdc-adh1* strains can be performed, and *C. acetobutylicum* Δ*cac15*Δ*upp*Δ*CAC2229::pdc-adh1* and *C. acetobutylicum* Δ*cac15*Δ*upp*Δ*CAC2499::pdc-adh1* strains sensitive to both erythromycin and thiamphenicol can be isolated.

The obtained strains *C. acetobutylicum* Δ*cac15*Δ*upp*Δ*CAC2229::pdc-adh1* and *C. acetobutylicum* Δ*cac15*Δ*upp*Δ*CAC2499::pdc-adhl* are characterized by having the pyruvate ferredoxin oxidoreductase (*pfor*) genes deleted and replaced by the synthetic *pdc-adh1* operon.

A similar strategy as provided above can also be used for the construction of a strain according to the present invention in which the pyruvate ferredoxin oxidoreductase (*pfor*) genes are deleted and replaced by the synthetic *pdc-adh1* operon and with reduced production of butyrate. This can be ensured by generating strains *C. acetobutylicum* Δ*cac15*Δ*upp*Δ*CAC2229::pdc-adh1*Δ*bk* and *C. acetobutylicum* Δ*cac15*Δ*upp*Δ*CAC2499::pdc-adhl*Δ*bk* which, in addition to the features of the strains described above have a deletion in the *bk* gene (CAC3075).

In further embodiments of the invention the strategy as described above is used for the construction of a strain according to the present invention in which the pyruvate ferredoxin oxidoreductase (*pfor*) genes are deleted and replaced by the synthetic *pdc-adhl* operon and with reduced production of butyrate and acetate. This can be ensured, e.g., by generating strains *C. acetobutylicum* Δ*cac15*Δ*upp*Δ*CAC2229::pdc-adhl*Δ*bk*Δ*pta-ak* and *C. acetobutylicum* Δ*cac15*Δ*upp*Δ*CAC2499 :pdc-adhl*Δ*bk*Δ*pta-ak* which, in addition to the features of the strains described above have a deletion in the *pta-ak* genes (CAC1742 and CAC1743).

In further particular embodiments of the invention, the strategy as described above is used for the construction of a strain according to the present invention in which the pyruvate ferredoxin oxidoreductase (*pfor*) genes are deleted and replaced by the synthetic *pdc-adhl* operon and with reduced production of butyrate, acetate, acetone and butanol. This can be ensured, e.g., by generating strains *C. acetobutylicum* Δ*cacl5*Δ*upp*Δ*CAC2229::pdc-adhl*Δ*bk*Δ*pta-ak*Δ*aad-ctfAB* and *C. acetobutylicum* Δ*cac15*Δ*upp*Δ*CAC2499::pdc-adh1*Δ*bk*Δ*pta-ak*Δ*aad-ctfAB* which, in addition to the features of the strains described above contain a deletion in the *aad-ctfAB.* Since on the pSOL1 plasmid of *C. acetobutylicum* ATCC 824, the *aad*/*adhe* gene (CAP0162) involved in butanol production is located directly upstream of the *ctfAB* genes (CAP0163-CAP0164) involved in acetone production, the deletion of both *aad*/*adhE* and *ctfAB* genes can be performed in one step leading to a decrease of both acetone and butanol productions.

In further particular embodiments of the invention, the strategy as described above is used for the construction of a strain according to the present invention in which the pyruvate ferredoxin oxidoreductase (*pfor*) genes are deleted and replaced by the synthetic *pdc-adh1* operon and with reduced production of butyrate, acetate, acetone, butanol and lactate. This can be ensured, e.g., by generating strains *C. acetobutylicum* Δ*cac15*Δ*upp*Δ*CAC2229::pdc-adh1*Δ*bk*Δ*pta-ak*Δ*aad-ctfAB*Δ*ldh* and *C. acetobutylicum* Δ*cac15*Δ*upp*Δ*CAC2499::pdc-adh1*Δ*bk*Δ*pta-ak*Δ*aad-ctfAB*Δ*ldh* which in addition to the features of the strains described above contain a deletion in the *Idh* gene (CAC0267).

In further particular embodiments, the combination of a mutation in the *aad*/*adhE, adhE2* and/or *bdhAB* nucleic acids and the introduction of at least one nucleic acid encoding an enzyme that converts pyruvate to acetaldehyde and at least one heterologous nucleic acid encoding an enzyme that converts acetaldehyde to ethanol leads to strains characterized by an increased production of ethanol.

In a further aspect, the present invention relates to recombinant Gram-positive *Clostridia* host cells for producing solvents, fuels and/or chemical intermediates, and preferably ethanol, from plant cell walls comprising:
(a) at least one nucleic acid encoding a plant cell wall degrading enzyme or a cellusomal scaffoldin, wherein said host cell is capable of expressing said nucleic acid and of producing and secreting said plant cell wall degrading enzyme or cellulosomal scaffoldin, and
(b) at least one nucleic acid encoding an enzyme that converts pyruvate to acetaldehyde and at least one nucleic acid encoding an enzyme that converts acetaldehyde to ethanol wherein said host cell is capable of expressing said nucleic acid, and,
(c) a mutation in at least one nucleic acid encoding for an enzyme in a metabolic pathway in said host cell, wherein said pathway produces an "undesirable" metabolite other than acetaldehyde from pyruvate or ethanol from acetaldehyde (or other than a relevant metabolite in the production of the solvent, fuel and/or chemical intermediate of interest by the organism) and wherein the mutation results in a reduced production of said "undesirable" metabolite.

In an embodiment of the present invention, the recombinant host cells may produce one or more solvents, fuels and/or chemical intermediates chosen from ethanol, acetone, butanol, isopropanol, propanol, 1,2 propanediol, propionic acid, butyric acid, ether and glycerine.

In particular embodiments, the recombinant host cells may produce, or may be engineered to produce, at least or mainly ethanol. The industrial importance of ethanol is rapidly increasing largely due to its utility as an environmentally acceptable fuel. Hence, in an embodiment, the recombinant host cell may be an ethanologenic microorganism.

According to this aspect of the invention, the inventors combine the introduction of a cellulase, complexes comprising one or more cellulases or capable of binding to one or more cellulases, originating from a Gram-positive or Gram-negative bacterium, to a Gram-positive solventogenic bacterium, with the engineering of the solventogenic metabolism of said bacterium. This combination has not previously been suggested and provides a unique recombinant solventogenic microorganism which enables a consolidated bioprocessing where the degradation of lignocellulosic materials and the fermentation of the degraded lignocellulose products for the production of ethanol is performed in a single process step by a single recombinant micro-organism.

Preferably, the recombinant host cell according to any aspect of the present invention is Gram-positive, such as for example a bacterium of the *Clostridium* species. In an embodiment, the recombinant solventogenic and preferably ethanologenic microorganism is a *Clostridium* species, preferably *Clostridium acetobutylicum.* In yet another embodiment, the recombinant solventogenic and preferably ethanologenic microorganism is *Clostridium beijerinckii.*

In a further aspect, the present invention relates to methods for the degradation of biomass comprising contacting said biomass with a recombinant host cell of the present invention.

In a further aspect, the present invention relates to methods for the degradation of biomass comprising contacting said biomass with a recombinant host cell of the present invention wherein said recombinant host cell provides cell wall degrading enzymes for the degradation of biomass.

In another aspect, the present invention relates to methods for the degradation of biomass and production of solvents, fuels and/or chemical intermediates comprising contacting said biomass with a recombinant host cell of the present invention wherein said recombinant host cell provides cell wall degrading enzymes for the degradation of biomass and a recombinant solventogenic metabolism.

In another aspect, the present invention relates to methods for the degradation of biomass and production of ethanol comprising contacting said biomass with a recombinant host cell of the present invention wherein said recombinant host cell provides cell wall degrading enzymes for the degradation of biomass and a recombinant ethanologenic metabolism.

Preferably, the biomass used in the methods of the present invention comprises plant biomass such as, but not limited to, cellulose, hemicellulose and/or lignin. Preferably, the cell wall degrading enzymes used in the methods of the present invention comprise enzymes such as, but not limited to, xylanases, cellulases, hemicellulases and lipolytic enzymes.

The methods of the present invention further provide that the production of solvents, fuels and/or chemical intermediates is done under anaerobic conditions.

As used in the present specification "anaerobic conditions" refers to conditions in which virtually all oxygen has been removed from the reaction medium, e.g., by passing nitrogen through the solution before the start of the reaction.

The present invention further relates to the use of a host cell comprising at least one nucleic acid encoding a plant cell wall degrading enzyme according to the present invention, wherein said host cell can be used for the degradation of biomass.

The present invention also relates to the use of a host cell according to the present invention comprising:
(a) at least one nucleic acid encoding a plant cell wall degrading enzyme or cellulosomal scaffoldin, wherein said host cell is capable of expressing said nucleic acid and of producing and secreting said plant cell wall degrading enzyme or cellulosomal scaffoldin, and/or
(b) at least one nucleic acid encoding an enzyme that converts pyruvate to acetaldehyde optionally associated with at least one heterologous nucleic acid encoding an enzyme that converts acetaldehyde to ethanol wherein said host cell is capable of expressing said nucleic acid, and/or,
(c) a mutation in at least one nucleic acid encoding for an enzyme in a metabolic pathway in said host cell, wherein said pathway produces a metabolite other than acetaldehyde from pyruvate or ethanol from acetaldehyde, and wherein said mutation results in a reduced production of said metabolite,
wherein said host cell can be used for the degradation of biomass and the production of solvents, fuels and/or chemical intermediates, and preferably ethanol, therefrom.

In particular embodiments, the invention relates to the use of a host cell according to the present invention comprising at least two of the elements (a), (b) and (c) recited above. In yet further particular embodiments the invention relates to the use of a host cell according to the present invention comprising all three of the elements (a), (b) and (c) recited above.

Particular embodiments of the present invention relate to the use of a host cell according to the present invention comprising:
(a) at least one nucleic acid encoding a plant cell wall degrading enzyme or cellulosomal scaffolding, wherein said host cell is capable of expressing said nucleic acid and of producing and secreting said plant cell wall degrading enzyme or cellulosomal scaffolding,
(b) at least one nucleic acid encoding an enzyme that converts pyruvate to acetaldehyde in association or not with at least one heterologous nucleic acid encoding an enzyme that converts acetaldehyde to ethanol wherein said host cell is capable of expressing said nucleic acid, and,
(c) a mutation in at least one nucleic acid encoding for an enzyme in a metabolic pathway in said host cell, wherein said pathway produces a metabolite other than acetaldehyde from pyruvate or ethanol from acetaldehyde, and wherein said mutation results in a reduced production of said metabolite,
wherein said host cell can be used for the production of ethanol.

The present invention also relates to the use of a host cell according to the present invention, wherein said host cell can be used for the production of solvents, fuels and/or chemical intermediates, such as ethanol.

The present invention also relates to kits and compositions comprising a host cell according to the present invention.

The nucleic acids used to modify the host can be introduced on a plasmid-based construction. It is not necessary that the nucleic acids encoding pyruvate decarboxylase and alcohol dehydrogenase activities be under common control. Chromosomal integration of heterologous *pdc* and *adh* nucleic acids can offer several advantages over plasmid-borne approaches, the latter having certain limitations for commercial processes.

To further improve the ethanol production yield, *C. acetobutylicum* metabolic engineering leading to the suppression of native side pathways producing undesired end-products is performed. Productions of butanol, acetone and lactic, butyric and acetic acids are prevented by chromosomal inactivation such as deletion, insertion and/or mutation of nucleic acids coding for an essential activity of their respective biosynthetic pathway.

In vivo evaluation of the *C. acetobutylicum* recombinant strains can be performed in discontinuous cultures by measuring the substrate to ethanol conversion yield, the substrate consumption rate, the ethanol production rate and selectivity.

### Examples

### Example 1:

### a) Construction of a recombinant strain of C. acetobutylicum secreting Cel5A from Clostridium cellulolyticum.

The DNA encoding the Cel5A was amplified while the restriction sites BamH1 and Nar1 were introduced at the 5' and 3' ends, respectively. After digestion with BamH1 and Nar1, the polynucleotide fragment was ligated to the pSOS952 vector (Perret et al. 2004. J. Bacteriol. 186: 253-257) digested by the same restriction endonucleases, thereby generating the p952-cel5A. The vector was subsequently methylated in vivo using the *E. coli* strain ER-2275(pAN1). The methylated vector was checked by sequencing and used to transform *C. acetobutylicum* by electropermeation. The secretion yield of Cel5A by the recombinant strain was estimated by monitoring the hydrolytic activity on amorphous cellulose and on CarboxyMethyl Cellulose. The secretion yield was approx. 5 mg/L.

### b) Construction of recombinant strain of C. acetobutylicum secreting Cel9M from Clostridium cellulolyticum.

The DNA encoding the Cel5A was amplified while the restriction sites BamH1 and Nar1 were introduced at the 5' and 3' ends, respectively. After digestion with BamH1 and Nar1, the polynucleotide fragment was ligated to the pSOS952 vector *(*Perret et al. 2004. J. Bacteriol. 186: 253-257*)* digested by the same restriction endonucleases, thereby generating the p952-cel9M. The vector was subsequently methylated in vivo using the *E. coli* strain ER-2275(pAN1). The methylated vector was checked by sequencing and used to transform *C. acetobutylicum* by electropermeation. The secretion yield of Cel9M by the recombinant strain is estimated by monitoring the hydrolytic activity on amorphous cellulose and on CarboxyMethyl Cellulose.

### Example 2:

### a) Construction of an artificial pdc-adh1 operon to express in Clostridium acetobutylicum

An artificial operon to express the heterologous pyruvate decarboxylase (*pdc*) gene of *Zymomonas mobilis* and the heterologous alcohol dehydrogenase (*adh1*) gene of *Saccharomyces cerevisiae* in *Clostridium acetobutylicum* was constructed.

To optimize the heterologous expression of the pyruvate decarboxylase *pdc* gene from the gram-negative bacterium *Zymomonas mobilis* subsp *mobilis* ZM4 in the gram-positive bacterium, *C. acetobutylicum,* a synthetic gene (table 3) was designed according to the preferred codon usage used in *C. acetobutylicum* (Karlin et al., 2004. PNAS 101:6182-6187). Upstream of the coding sequence, the clostridial RBS (AGGAGG) was introduced, while the restriction sites BamH1 and SfoI were inserted at the 5' and 3' ends, respectively. After digestion with BamH1 and Sfo1, the polynucleotide fragment was ligated to the pSOS95 vector (GenBank accession number AY187686) digested with the same endonucleases to yield the pSOS95-pdc vector. The ADH1 gene (locus tag YOL086C) was PCR-amplified from *Saccharomyces cerevisiae* S288C genomic DNA using primers ADH_D and ADH_R (table 4) introducing a SfoI site at both ends. The amplified fragment was digested by SfoI and ligated to the pSOS95-pdc linearized with SfoI creating the pSOS95-pdcadh1 vector.

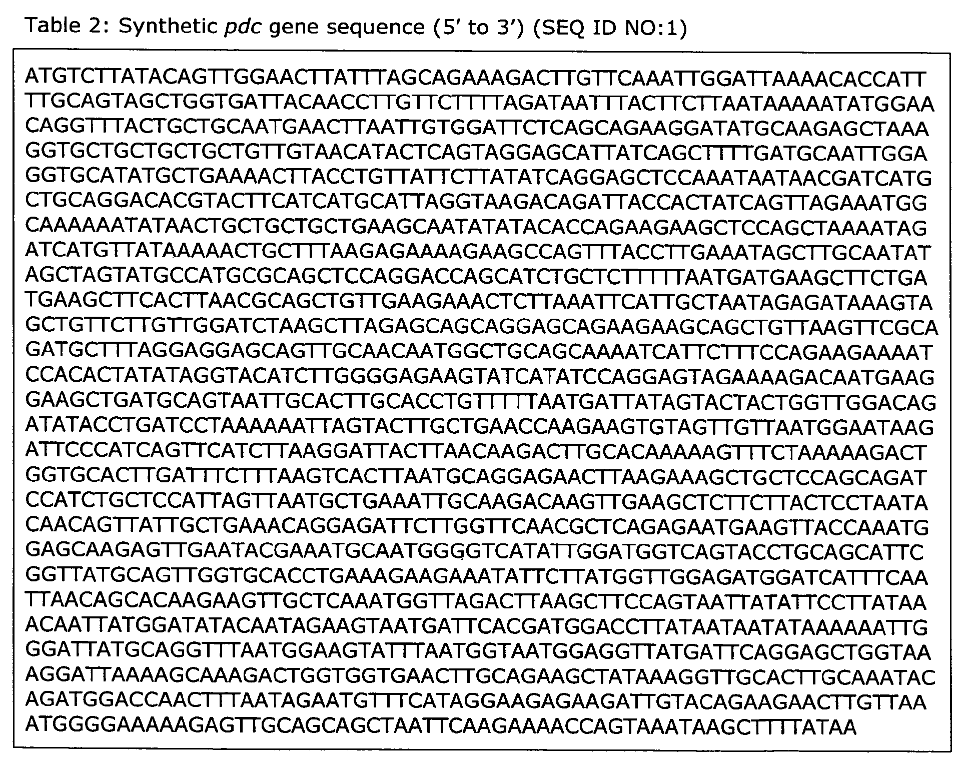

**Table 3: Primer sequences**

| Name | Primer sequence (5' to 3') |
|---|---|
| ADH_D | AAAAGGCGCCGGGAGGATAAACATGTCTATCCCAGAAACTCAAAAAGGTG (SEQ ID NO:2) |
| ADH_R | AAAAGGCGCCTTATTTAGAAGTGTCAACAACGTATCTACC (SEQ ID NO:3) |

### b) Construction of a C. acetobutylicum strain expressing the synthetic pdc gene of Z. mobilis.

Expression of the *Z. mobilis pdc* gene in a plasmid borne manner was achieved by the electro-transformation of *Clostridium* strains with the pSOS95-pdc vector described in example 2. Transformants were selected on Petri dishes for resistance to erythromycin (40 µg ml⁻¹). After extraction, the vectors were validated by sequencing.

As a result, *C. acetobutylicum* (pdc⁺) strains were capable to convert pyruvate to acetaldehyde via the expression of a pyruvate decarboxylase activity.

### Example 3:

Construction of *C. acetobutylicum* strains expressing the artificial *pdc-adhl* operon as constructed in example 2.

Expression of the artificial *pdc-adh1* operon in a plasmid borne manner was achieved by the electro-transformation of *Clostridium* strains with the pSOS95-pdcadh1 vector described in example 2. Transformants were selected on Petri dishes for resistance to erythromycin (40 µg ml⁻¹). After extraction, the vectors were validated by sequencing.

Expression of the artificial *pdc-adhl* operon via a chromosomal insertion strategy is achieved by specific insertion of the artificial *pdc-adhl* operon in a targeted chromosomal gene/sequence by single crossing-over with a non-replicative plasmid lacking an origin of replication for Clostridium (Green et al., 1996, Microbiology, 142:2079-2086, Sellers et al., 2008, Metab. Eng. doi:10.1016/j.ymben.2008.07.005). Integrants are selected on Petri dishes for resistance to erythromycin (40 µg ml⁻¹). When antibiotic resistance needs to be removed to allow additional chromosomal mutation(s) to be performed in the same Clostridium strain, the chromosomal insertion of the synthetic *pdc-adh1* operon is achieved by specific insertion of the synthetic *pdc-adhl* operon in a targeted chromosomal gene/sequence by a double crossover integration through homologous recombination of a replicative integrative plasmid and subsequent removal of the antibiotic resistance gene with the Flp recombinase system from Saccharomyces cerevisiae (technique described in PCT/EP2006/066997).

As a result, *C. acetobutylicum* (pdc⁺ adh1⁺) strains are obtained which are capable of converting pyruvate to acetaldehyde and acetaldehyde to ethanol via expressions of pyruvate decarboxylase and alcohol dehydrogenase activities.

### Example 4:

The present example provides in the construction of *C. acetobutylicum* strains according to the present invention with reduced production of acetyl coenzyme A, butyrate, acetate, acetone, butanol and/or lactate.

Molecular techniques for multiple unmarked mutations in the same *C. acetobutylicum* strain are applied to mutate one or more of the *pfor, bk, pta-ak, ctfAB, aad*/*adhE* and/or *Idh* genes listed below:
- pyruvate ferredoxin oxidoreductase (*pfor*). Sequence analysis of the *C. acetobutylicum* ATCC 824 genome (Nölling et al., 2001, J. Bacteriol. 183:4823-4838) showed two open reading frames CAC2229 and CAC2499 encoding putative pyruvate ferredoxin oxidoreductase: they exhibit high amino acid sequence homology and identity with the pyruvate ferredoxin oxidoreductase of *Desulfovibrio africanus* (CAC2229: identity 54%, homology 67% and CAC2499: identity 53%, homology 66%) to inhibit the conversion of pyruvate into acetyl coenzyme.
- lactate dehydrogenase (*Idh*) enzyme encoded by the CAC0267 gene in *C. acetobutylicum* ATCC 824 genome sequence (Nölling et al., 2001, J. Bacteriol. 183:4823-4838) to inhibit the conversion of pyruvate into lactate.
- phosphotransacetylase (*pta*) and acetate kinase (*ak*) encoded respectively by CAC1742 and CAC1743 in C. *acetobutylicum* ATCC 824 genome sequence (Nölling et al., 2001, J. Bacteriol. 183:4823-4838) to inhibit the conversion of acetyl coenzyme A into acetate.
- acetyl-coenzyme A acetyltransferase or thiolase (*thlA*)*,* a β-hydroxybutyryl coenzyme A dehydrogenase (*hbd*), a crotonase (*crt*), a butyryl coenzyme A dehydrogenase (*bcd*) encoded respectively by CAC2873, CAC2708, CAC2712 and CAC2711 nucleic acids in *C. acetobutylicum* ATCC 824 genome sequence (Nölling et al., 2001, J. Bacteriol. 183:4823-4838) to inhibit the conversion of acetyl coenzyme A into butyryl coenzyme A.
- A transferase (*ctfAB*) and an acetoacetate decarboxylase (*adc*) encoded respectively by CAP0163-CAP0164 and CAP165 in *C. acetobutylicum* ATCC 824 genome sequence (Nölling et al., 2001, J. Bacteriol. 183:4823-4838) to inhibit the conversion of acetoacetyl coenzyme A into acetone.
- phosphotransbutyrylase (*ptb*) and a butyrate kinase (*bk*) encoded respectively by CAC3076 and CAC3075 in *C. acetobutylicum* ATCC 824 genome sequence (Nölling et al., 2001, J. Bacteriol. 183:4823-4838) to inhibit conversion of butyryl coenzyme A into butyrate.
- a bifunctional butyraldehyde-butanol dehydrogenase (*aad*/*adhE, adhE2*) and a butanol dehydrogenase (*bdhAB*) encoded respectively by CAP0162, CAP0035, CAC3298 and CAC3299 in *C. acetobutylicum* ATCC 824 genome sequence (Nölling et al., 2001, J. Bacteriol. 183:4823-4838) to inhibit the conversion of butyryl coenzyme A into butanol.

Mutants are first selected by acquisition of resistance to erythromycin. The antibiotic resistance marker flanked by FRT sites is subsequently removed by expression of FLP recombinase of *S. cerevisiae.* Correct inactivation is checked by PCR or gene sequencing.

### Example 5:

Construction of a *C. acetobutylicum* strain capable to convert pyruvate to acetaldehyde and acetaldehyde to ethanol via heterologous expressions of pyruvate decarboxylase and alcohol dehydrogenase activities and with reduced production of acetyl coenzyme A, butyrate, acetate, acetone, butanol and/or lactate.

Combination of techniques described in examples 2 to 4 are used to generate a *C. acetobutylicum* strain according to the present invention.

### Example 6:

Construction of a *C. acetobutylicum* strain capable to convert cellulosic substrate into pyruvate, pyruvate into acetaldehyde, acetaldehyde into ethanol and with reduced production of acetyl coenzyme A, butyrate, acetate, acetone, butanol and/or lactate.

Combination of techniques described in examples 1 to 5 are used to generate a *C. acetobutylicum* strain according to one aspect of the present invention.

### Example 7:

Fermentation on glucose or cellulose substrates of the *C. acetobutylicum* strains, including reference strains and strains as constructed in the above examples.

The following methods are used:
1) method 1
   - The *C. acetobutylicum* strains are first cultivated (inoculation at 10% v/v) in 30 ml anaerobic flask in glucose synthetic medium (Soni et al., 1987, Appl. Microbiol. Biotechnol. 27:1-5) supplemented with 40 mM acetic acid and for recombinant strains with 40 µg ml⁻¹ erythromycin.
   - *C*. *acetobutylicum* strains were subsequently cultured in 1.5 l batch reactors (inoculation at 10% v/v) on glucose minimal medium (Vasconcelos et al., 1994, J. Bacterial. 176:1443-1450) supplemented for recombinant strains with 40 µg ml⁻¹ erythromycin. The cultures were maintained under nitrogen at 37°C and pH 4.8.
2) method 2
   - The *C. acetobutylicum* strains were first cultivated (inoculation at 10% v/v) in 30 ml anaerobic flask in cellobiose (5 g/L) rich medium (containing 16 g of bacto tryptone/liter 10 g of yeast extract/liter and 4 g of NaCl/liter) supplemented for recombinant strains with 40 µg ml⁻¹ erythromycin.
   - *C. acetobutylicum* strains were subsequently cultured in 1.5 l batch reactors (inoculation at 10% v/v) on rich medium supplemented with amorphous cellulose (3 g/L) and for recombinant strains with 40 µg ml⁻¹ erythromycin. The cultures were maintained under nitrogen at 37°C and pH 5.5.

### Example 8:

Determination of the substrate to ethanol conversion yield, the substrate consumption rate, the ethanol production rate and selectivity of the *C*. *acetobutylicum* strains *C*. *acetobutylicum* ATCC 824 (pPHIMP2) and *C. acetobutylicum* ATCC 824 (pSOS95-pdc).

The strains were cultivated according to method 1 of Example 7.

After 7 day incubation at 37 °C for culture on glucose in anaerobic flask or 36 hour in reactors, glucose, organic acids and solvents were quantified by HPLC, the separation was obtained with an Aminex HPX-87H column. Elution was done at 25°C with 0.031 mM H₂SO₄ at a flow rate of 0.7 ml min⁻¹.

For the growth on amorphous cellulose in reactor, aliquots of the culture were prepared at specific intervals and analyzed for cellulose consumption and bacterial growth. For cellulose consumption, the sample was centrifuged and the pellet containing the residual cellulose and the bacterial cells was hydrolyzed in 12 M H₂SO₄, for one hour at 37°C. The suspension was subsequently diluted 10 times in distilled water and heated at 120°C for one hour. The sample was cooled down and the pH was adjusted to 6-8 using sodium hydroxide. The glucose content was determined by high performance anion exchanger chromatography coupled with pulsed amperometric detection (HPAEC-PAD) using an ICS-3000 ion chromatography system (Dionex). 5 to 25 µL of the diluted samples were applied to a Dionex CarboPac PA1 column (4 x 250 mm) equipped with the corresponding guard column (4 x 50 mm) at 30°C. Sugars were eluted with the buffers 0.1 M NaOH and 0.5 M sodium acetate + 0.1 M NaOH as the eluents A and B, respectively, using the following multi-steps procedure: an isochratic separation was performed during the first 5 minutes using 95 % A + 5% B as the eluent. This first step was followed by a linear gradient from 10 to 37 % B during 8 minutes. The column was then washed for two minutes with 99 % B, and subsequently equilibrated with the initial eluent (95 % A + 5 % B) for another 2.5 minutes. The flow rate was kept at 1 mL/min.

Cell density was determined as follow: the culture sample was centrifuged and the pellet that contains the residual cellulose and the bacterial cells was washed four times with distilled water. The total protein content of the last pellet was determined using the method of Lowry (Lowry et al., 1951. J. Biol. Chem. 193:265-275)

Evolutions over the time of the substrate and fermentation end-product concentrations allow the calculations of the physiological characteristics of the *C. acetobutylicum* strains of interest (Table 4).

**Table 4:**

| Strains | Culture | substrate | Yield (mol ethanol/mol substrate) ± SD | ethanol selectivity (% mol ethanol/mol solvent) |
|---|---|---|---|---|
| *C. acetobutylicum* ATCC 824 (pPHIMP2) | anaerobic flask | glucose | 0.13 ± 0.04 | 12.2 |
| | reactor | glucose | | |
| *C*. *acetobutylicum* ATCC 824 (pSOS95-pdc) | anaerobic flask | glucose | 0.29 ± 0.03 | 29.8 |
| | reactor | glucose | | |

Table 4 shows that expression of the synthetic *pdc* gene in strain *C*. *acetobutylicum* ATCC 824 (pSOS95-pdc) cultured in anaerobic flask led to a 2.2 fold increased yield of glucose conversion into ethanol and a 2.4 fold increase of the ethanol selectivity. The *C. acetobutylicum* ATCC 824 (pPHIMP2) is a reference strain containing the pPHIMP2 plasmid with does not express any catalytic enzyme. SD: standard deviation.

## Claims

1. A recombinant Gram-positive *Clostridia* host cell comprising:
(a) at least one heterologous nucleic acid encoding a plant cell wall degrading enzyme, wherein said host cell is capable of expressing said nucleic acid and of producing and secreting said plant cell wall degrading enzyme; and/or
(b) at least one nucleic acid encoding an enzyme that converts pyruvate to acetaldehyde optionally combined with at least one heterologous nucleic acid encoding an enzyme that converts acetaldehyde to ethanol wherein said host cell is capable of expressing said nucleic acid;
and/or,
(c) a mutation in at least one nucleic acid encoding for an enzyme in a metabolic pathway in said host cell, wherein said pathway produces a metabolite other than acetaldehyde from pyruvate or ethanol from acetaldehyde, and wherein said mutation results in a reduced production of said metabolite.

2. The recombinant Gram-positive *Clostridia* host cell according to claim 1 comprising:
(b) at least one nucleic acid encoding an enzyme that converts pyruvate to acetaldehyde optionally combined with at least one heterologous nucleic acid encoding an enzyme that converts acetaldehyde to ethanol wherein said host cell is capable of expressing said nucleic acid;
and,
(c) a mutation in at least one nucleic acid encoding for an enzyme in a metabolic pathway in said host cell, wherein said pathway produces a metabolite other than acetaldehyde from pyruvate or ethanol from acetaldehyde, and wherein said mutation results in a reduced production of said metabolite.

3. The recombinant *Clostridia* host cell according to any one of claims 1 or 2, comprising at least one heterologous nucleic acid encoding one or more plant cell wall degrading enzymes, wherein said cell wall degrading enzymes are cellulases.

4. The recombinant *Clostridia* host cell according to any one of claims 1 to 3, comprising at least one heterologous nucleic acid encoding one or more plant cell wall degrading enzymes, wherein said cell wall degrading enzymes are cellulases appended with appropriate dockerin domains or modules and at least one heterologous nucleic acid encoding a wild-type or hybrid scaffoldin bearing the cognate cohesin domains or modules, wherein said host cell is capable of expressing said nucleic acids and produce extracellularly a cellulosome composed of said cellulases bound to said scaffoldin.

5. The host cell according to any of claims 1 to 4, wherein said host cell comprises a recombinant solventogenic metabolism adapted to increase the production of solvents, fuels and/or chemical intermediates.

6. The host cell according to claim 5, wherein said recombinant solventogenic metabolism comprises at least one nucleic acid encoding an enzyme that converts pyruvate to acetaldehyde optionally associated with at least one heterologous nucleic acid encoding an enzyme that converts acetaldehyde to ethanol wherein said host cell is capable of expressing said nucleic acid(s).

7. The host cell according to claim 6, wherein said nucleic acid encoding an enzyme that converts pyruvate to acetaldehyde is pyruvate decarboxylase (*pdc*)

8. The host cell according to any of claims 1 to 7, wherein said recombinant solventogenic metabolism comprises a mutation in at least one nucleic acid encoding for an enzyme in a metabolic pathway in said host cell, wherein said pathway produces a metabolite other than acetaldehyde from pyruvate or ethanol from acetaldehyde.

9. The host cell according to claim 8, wherein said mutation is a mutation in at least one nucleic acid encoding an enzyme chosen from the group comprising pyruvate ferredoxin oxidoreductase (*pfor*)*,* phosphotransacetylase (*pta*)*,* acetate kinase (*ak*)*,* coenzym A transferase (*ctfAB*)*,* acetoacetate decarboxylase (*adc*), phosphotransbutyrylase (*ptb*), butyrate kinase (*bk*)*,* lactate dehydrogenase (*Idh*)*,* thiolase *(thl),* β-hydroxybutyryl coenzyme A dehydrogenase (*hbd*), crotonase (*crt*), butyryl coenzyme A dehydrogenase (*bcd*)*,* bifunctional butyraldehyde-butanol dehydrogenase (*aad*/*adhE, adhE2*) and/or butanol dehydrogenase (*bdhAB*).

10. The host cell according to claims 8 or 9, comprising a mutation in at least one nucleic acid encoding an enzyme in the metabolic pathway that converts pyruvate to lactate.

11. The host cell according to claim 9 comprising a mutation in at least one nucleic acid encoding an enzyme in the metabolic pathway that converts pyruvate to acetyl coenzyme A, which is a mutation in the nucleic acid encoding pyruvate ferredoxin oxidoreductase (*pfor*).

12. The host cell according to any one of claims 9 to 11, comprising a mutation in at least one nucleic acid encoding an enzyme in the metabolic pathway that converts acetyl coenzyme A to acetate.

13. The host cell according to any of claims 9 to 12, comprising a mutation in at least one nucleic acid encoding an enzyme in the metabolic pathway that converts acetoacetyl coenzyme A to acetone.

14. The host cell according to any of claims 9 to 13, comprising a mutation in at least one nucleic acid encoding an enzyme in the metabolic pathway that converts butyryl coenzyme A to butyrate.

15. The recombinant *Clostridia* host cell according to any one of claims 1 to 14, wherein said host cell is a Clostridium species selected from the group comprising *Clostridium acetobutylicum and C. beijerinckii.*

16. A method for degradation of biomass comprising contacting said biomass with a recombinant host cell according to any of claims 1 to 15.

17. Use of a recombinant host cell according to any of claims 1 to 15 for the degradation of biomass.

18. Use of recombinant a host cell according to any of claims 1 to 15 for the production of solvents, fuels and/or chemical intermediates, and preferably ethanol, and the degradation of biomass.

19. A kit composition comprising a host cell according to any of claims 1 to 15.
